# EUROPEAN PATENT APPLICATION

(11) **EP 4 364 739 A1**
(43) Date of publication of application: **08.05.2024**
(21) Application number: 22832976.9
(22) Date of filing: 22.06.2022
(51) Int. Cl.: A61K 31/496, A61K 31/435, A61K 31/454, A61K 31/495, A61K 31/5377, A61K 31/55, A61P 13/12, A61P 43/00

(54) **PHARMACEUTICAL COMPOSITION FOR PREVENTING AND/OR TREATING KIDNEY INJURY, AND AUTOPHAGY ACTIVATOR**

(30) Priority: 29.06.2021 JP 2021107743
(71) Applicant: OSAKA UNIVERSITY, Suita-shi Osaka 565-0871 (JP); Kyoto University, Kyoto-shi, Kyoto 606-8501 (JP)
(72) Inventor: NAGATA, Ryu, Suita-shi, Osaka 565-0871 (JP); ISAKA, Yoshitaka, Suita-shi, Osaka 565-0871 (JP); YAMAMOTO, Takeshi, Suita-shi, Osaka 565-0871 (JP); YONISHI, Hiroaki, Suita-shi, Osaka 565-0871 (JP); YOSHIMORI, Tamotsu, Suita-shi, Osaka 565-0871 (JP); HAMASAKI, Maho, Suita-shi, Osaka 565-0871 (JP); MORI, Yasuo, Kyoto-shi, Kyoto 606-8501 (JP)
(74) Representative: Studio Torta S.p.A.
(86) International application number: PCT/JP2022/024923
(87) International publication number: WO 2023/276828

(57) **Abstract**

An object of this invention is to provide a pharmaceutical composition for preventing and/or treating acute kidney injury, and an autophagy activator. The invention relates to, for example, a pharmaceutical composition for preventing and/or treating acute kidney injury, comprising a compound represented by formula (1), a salt thereof, or a prodrug thereof: 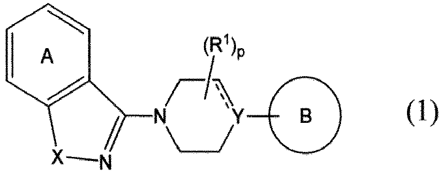 wherein
A is an optionally substituted benzene ring;
B is an optionally substituted aryl or an optionally substituted heteroaryl;
X is an oxygen atom or a sulfur atom;
Y is a nitrogen atom or a carbon atom;
- - - - - - of - - - - -Y is a single or double bond when Y is a carbon atom, or
- - - - - - of - - - - -Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

## Description

### Technical Field

The present invention relates to a pharmaceutical composition for preventing and/or treating kidney injury, and to an autophagy activator.

### Background Art

With the aging of the population and increase in lifestyle-related diseases, the number of patients with chronic kidney disease (CKD), end-stage renal disease (ESRD), and dialysis is increasing every year, becoming a medical and social problem. Under such circumstances, onconephrology, which focuses on the relationship between kidneys and cancer, has recently been attracting attention as a new field of nephrology. In Japan alone, the number of prescriptions for platinum-containing drugs (platinum-based preparations) as an anticancer drug exceeds 1 million per year. Cisplatin, in particular, is widely used in the treatment of various malignant tumors, including head and neck, lung, testicular, ovarian, and bladder cancers. However, cisplatin has adverse effects, such as kidney injury. Since kidney injury, in particular, occurs at low doses, the administration amount is limited mainly due to nephrotoxicity. Cisplatin is partly filtered out of the glomerulus, and is partly secreted from the proximal tubules and excreted into the urine; thus, the concentration of cisplatin in the deep cortical layer is several times higher than in other organs. Kidney injury is found in the proximal tubules of kidneys and clinically can cause pathological conditions of acute tubular necrosis. Accordingly, the use of cisplatin causes acute kidney injury (AKI) at a high frequency; however, sufficient prevention and treatment methods have not yet been established. Thus, if repeated AKI due to cisplatin administration develops CKD, and if kidney function does not recover sufficiently, a reduction in the amount of cisplatin administration will be necessary, making it difficult to continue the treatment of malignant tumors and worsening the life prognosis of cancer-bearing patients.

"Autophagy" is the general term for degradation of cytoplasmic components in lysosomes. Autophagy in kidney diseases has been studied and reported to play a protective role against AKI caused by ischemia reperfusion injury, AKI caused by cisplatin administration, and CKD due to aging or obesity (Non-patent Literature (NPL) 1 to 3). More importantly, although the role of autophagy becomes more important in CKD, if the late stage in the lysosomal system in autophagy stagnates, resistance to the disease will be insufficient, and the pathological conditions will be worsened; given this, the concept of "autophagy stagnation" has been proposed (NPL 4 to 6). There has also been a report on search for food components that release the stagnation of autophagy, which revealed that eicosapentaenoic acid (EPA) attenuates renal lipotoxicity by restoring autophagy (NPL 7). Autophagy deficiency has been reported to be involved not only in kidney diseases but also in many diseases, such as Parkinson's disease and diabetes. Therefore, there is a worldwide need to search for and develop drugs that enhance autophagy.

### Citation List

### Non-patent Literature

NPL 1: Kimura T, Takabatake Y, Takahashi A, Kaimori JY, Matsui I, Namba T, Kitamura H, Niimura F, Matsusaka T, Soga T, Rakugi H, Isaka Y. Autophagy protects the proximal tubule from degeneration and acute ischemic injury. J Am Soc Nephrol. 2011 May; 22(5):902-13. doi: 10.1681/ASN.2010070705. Epub 2011 Apr 14. PMID: 21493778; PMCID: PMC3083312.
NPL 2: Takahashi A, Kimura T, Takabatake Y, Namba T, Kaimori J, Kitamura H, Matsui I, Niimura F, Matsusaka T, Fujita N, Yoshimori T, Isaka Y, Rakugi H. Autophagy guards against cisplatin-induced acute kidney injury. Am J Pathol. 2012 Feb; 180(2):517-25. doi: 10.1016/j.ajpath.2011.11.001. PMID: 22265049.
NPL 3: Namba T, Takabatake Y, Kimura T, Takahashi A, Yamamoto T, Matsuda J, Kitamura H, Niimura F, Matsusaka T, Iwatani H, Matsui I, Kaimori J, Kioka H, Isaka Y, Rakugi H. Autophagic clearance of mitochondria in the kidney copes with metabolic acidosis. J Am Soc Nephrol. 2014 Oct; 25(10):2254-66. doi: 10.1681/ASN.2013090986. Epub 2014 Apr 3. PMID: 24700866; PMCID: PMC4178439.
NPL 4: Yamamoto T, Takabatake Y, Kimura T, Takahashi A, Namba T, Matsuda J, Minami S, Kaimori JY, Matsui I, Kitamura H, Matsusaka T, Niimura F, Yanagita M, Isaka Y, Rakugi H. Time-dependent dysregulation of autophagy: Implications in aging and mitochondrial homeostasis in the kidney proximal tubule. Autophagy. 2016 May 3; 12(5):801-13. doi: 10.1080/15548627.2016.1159376. Epub 2016 Mar 17. PMID: 26986194; PMCID: PMC4854554.
NPL 5: Yamamoto T, Takabatake Y, Takahashi A, Kimura T, Namba T, Matsuda J, Minami S, Kaimori JY, Matsui I, Matsusaka T, Niimura F, Yanagita M, Isaka Y. High-Fat Diet-Induced Lysosomal Dysfunction and Impaired Autophagic Flux Contribute to Lipotoxicity in the Kidney. J Am Soc Nephrol. 2017 May; 28(5):1534-1551. doi: 10.1681/ASN.2016070731. Epub 2016 Dec 8. PMID: 27932476; PMCID: PMC5407727.
NPL 6: Takabatake Y, Yamamoto T, Isaka Y. Stagnation of autophagy: A novel mechanism of renal lipotoxicity. Autophagy. 2017 Apr 3; 13(4):775-776. doi: 10.1080/15548627.2017.1283084. Epub 2017 Feb 6. PMID: 28165842; PMCID: PMC5388212.
NPL 7: Yamamoto T, Takabatake Y, Minami S, Sakai S, Fujimura R, Takahashi A, Namba-Hamano T, Matsuda J, Kimura T, Matsui I, Kaimori JY, Takeda H, Takahashi M, Izumi Y, Bamba T, Matsusaka T, Niimura F, Yanagita M, Isaka Y. Eicosapentaenoic acid attenuates renal lipotoxicity by restoring autophagic flux. Autophagy. 2020 Jun 28: 1-14. doi: 10.1080/15548627.2020.1782034. Epub ahead of print. PMID: 32546086.

### Summary of Invention

### Technical Problem

An object is to provide a pharmaceutical composition for preventing and/or treating acute kidney injury, e.g., kidney injury caused by administration of a platinum-containing drug, and provide an autophagy activator.

### Solution to Problem

To solve the above problem, the present inventors conducted extensive research and consequently found that a compound represented by formula (1), a compound represented by formula (2), a salt thereof, or a prodrug thereof, described below, can attenuate acute kidney injury, e.g., kidney injury caused by administration of a platinum-containing drug, and can activate autophagy in cells. The present invention has thus been accomplished. Representative inventions of the present invention are as follows.

### Item 1.

A pharmaceutical composition for preventing and/or treating acute kidney injury, comprising a compound represented by formula (1), a salt thereof, or a prodrug thereof: 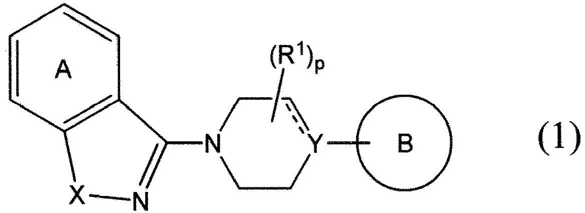 wherein
A is an optionally substituted benzene ring;
B is an optionally substituted aryl or an optionally substituted heteroaryl;
X is an oxygen atom or a sulfur atom;
Y is a nitrogen atom or a carbon atom;
------ of -----Y is a single or double bond when Y is a carbon atom, or
------ of -----Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

### Item 2.

The pharmaceutical composition according to Item 1, wherein in formula (1),
B is an optionally substituted monocyclic aryl or an optionally substituted monocyclic or bicyclic nitrogen-containing heteroaryl.

### Item 3.

The pharmaceutical composition according to Item 1 or 2, wherein in formula (1),
A is a benzene ring optionally substituted with at least one substituent selected from the group consisting of the following A-1 to A-16:
A-1) halogen,
A-2) hydroxyl,
A-3) nitro,
A-4) cyano,
A-5) carboxyl,
A-6) optionally substituted amino,
A-7) optionally substituted cyclic amino,
A-8) optionally substituted lower alkyl,
A-9) optionally substituted lower alkoxy,
A-10) lower alkoxycarbonyl,
A-11) lower alkylsulfonyl,
A-12) carbamoyl optionally substituted with lower alkyl or lower alkylsulfonyl,
A-13) optionally substituted cyclic aminocarbonyl,
A-14) sulfamoyl optionally substituted with lower alkyl,
A-15) optionally substituted cyclic aminosulfonyl, and
A-16) tetrazolyl.

### Item 4.

The pharmaceutical composition according to any one of Items 1 to 3, wherein in formula (1),
B is a monocyclic aryl or a monocyclic or bicyclic heteroaryl, the monocyclic aryl is optionally substituted with at least one substituent selected from the group consisting of the following B-1 to B-16, and
the monocyclic or bicyclic heteroaryl is optionally substituted with at least one substituent selected from the group consisting of the following B-1 to B-17:
   B-1) halogen,
   B-2) hydroxyl,
   B-3) nitro,
   B-4) cyano,
   B-5) carboxyl
   B-6) optionally substituted amino,
   B-7) optionally substituted cyclic amino,
   B-8) optionally substituted lower alkyl,
   B-9) optionally substituted lower alkoxy,
   B-10) lower alkoxycarbonyl,
   B-11) lower alkylsulfonyl,
   B-12) carbamoyl optionally substituted with lower alkyl or lower alkylsulfonyl,
   B-13) optionally substituted cyclic aminocarbonyl,
   B-14) sulfamoyl optionally substituted with lower alkyl,
   B-15) optionally substituted cyclic aminosulfonyl,
   B-16) tetrazolyl, and
   B-17) oxo.

### Item 5.

The pharmaceutical composition according to any one of Items 1 to 4, wherein in formula (1), the benzisoxazole or benzisothiazole skeleton is substituted at the 4-position.

### Item 6.

The pharmaceutical composition according to any one of Items 1 to 3, wherein in formula (1),
B is substituted pyridyl or substituted phenyl, and at least one of the carbon atoms in ortho-positions with respect to the Y-bound carbon atom on the pyridine or benzene ring is substituted.

### Item 7.

The pharmaceutical composition according to any one of Items 1 to 4, wherein in formula (1),
A is a benzene ring optionally substituted with at least one substituent selected from the group consisting of halogen, lower alkoxy, and optionally halogen-substituted lower alkyl;
B is pyridyl or phenyl each optionally substituted with at least one substituent selected from the group consisting of the following B-1, B-5, B-8, B-10, B-12, and B-13:
   B-1) halogen,
   B-5) carboxyl,
   B-8) optionally substituted lower alkyl,
   B-10) lower alkoxycarbonyl,
   B-12) carbamoyl optionally substituted with lower alkyl or lower alkylsulfonyl, and
   B-13) optionally substituted cyclic aminocarbonyl; and
   each R¹ independently represents C₁-C₃ alkyl, or two R¹s are bound to each other to form a methylene group, a dimethylene group, or a trimethylene group, or
   (R¹)ₚ is oxo.

### Item 8.

The pharmaceutical composition according to any one of Items 1 to 4 and 7, wherein the compound represented by formula (1) is a compound represented by formula (1A): 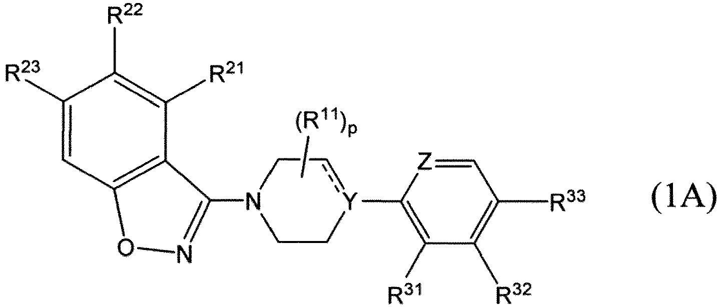 wherein
Z is a nitrogen atom or CH;
Y is a nitrogen atom or a carbon atom;
------ of -----Y is a single or double bond when Y is a carbon atom, or ------ of -----Y is a single bond when Y is a nitrogen atom;
each R¹¹ independently represents methyl or ethyl, or
two R¹¹s may be bound to each other to form a crosslinked structure by methylene, dimethylene, or trimethylene;
p is 0, 1, or 2; or
(R¹¹)ₚ is oxo;
R²¹, R²², and R²³ independently represent a hydrogen atom, halogen, carbamoyl, or trifluoromethyl; and
R³¹, R³², and R³³ independently represent a hydrogen atom, halogen, halogen-substituted lower alkyl, methyl, carboxyl, lower alkoxycarbonyl, monomethylaminocarbonyl, or dimethylaminocarbonyl.

### Item 9.

The pharmaceutical composition according to Item 8, wherein in formula (1A),
R²¹ is a chlorine atom or trifluoromethyl,
R²² and R²³ are each a hydrogen atom,
R³¹ is a chlorine atom,
R³² is a hydrogen atom, and
R³³ is a hydrogen atom, carboxyl, or lower alkoxycarbonyl.

### Item 10.

The pharmaceutical composition according to any one of Items 1 to 4, wherein the compound represented by formula (1) is Compound 011, Compound 021, Compound 031, Compound 041, Compound 061, Compound 071, Compound 081, Compound 091, Compound 101, Compound 111, Compound 121, Compound 131, Compound 141, Compound 151, Compound 161, Compound 171, Compound 191, Compound 221, Compound 281, Compound 311, Compound 321, Compound 331, Compound 341, Compound 351, Compound 361, Compound 371, Compound 381, Compound 391, Compound 401, or Compound 431, each represented by any of the following structures:

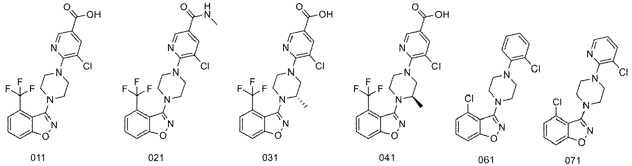

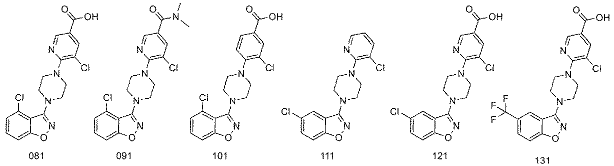

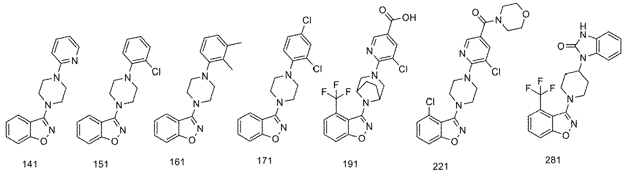

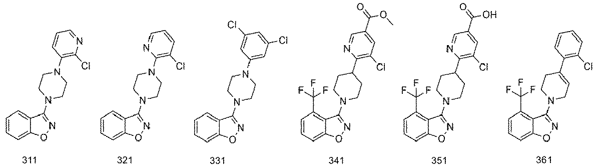

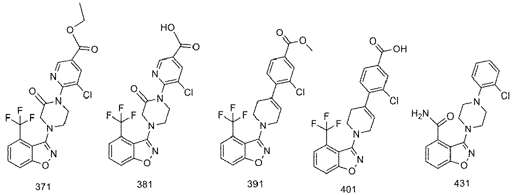

### Item 11.

A pharmaceutical composition for preventing and/or treating acute kidney injury, comprising a compound represented by formula (2), a salt thereof, or a prodrug thereof: 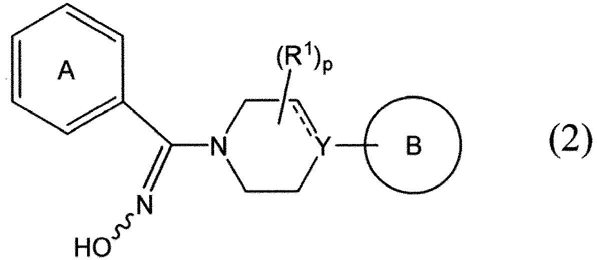 wherein
A is an optionally substituted benzene ring;
B is an optionally substituted aryl or an optionally substituted heteroaryl;
Y is a nitrogen atom or a carbon atom;
------ of -----Y is a single or double bond when Y is a carbon atom, or
------ of -----Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

### Item 12.

The pharmaceutical composition according to any one of Items 1 to 11, which is for oral administration.

### Item 13.

The pharmaceutical composition according to any one of Items 1 to 12, wherein the acute kidney injury is kidney injury caused by administration of a platinum-containing drug.

### Item 14.

The pharmaceutical composition according to Item 13, wherein the platinum-containing drug is cisplatin.

### Item 15.

An autophagy activator comprising a compound represented by formula (1), a salt thereof, or a prodrug thereof: 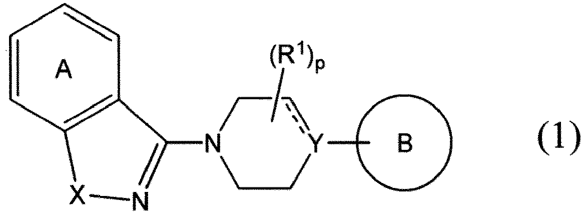 wherein
A is an optionally substituted benzene ring;
B is an optionally substituted aryl or an optionally substituted heteroaryl;
X is an oxygen atom or a sulfur atom;
Y is a nitrogen atom or a carbon atom;
------ of -----Y is a single or double bond when Y is a carbon atom, or
------ of -----Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

### Item 16.

An autophagy activator comprising a compound represented by formula (2), a salt thereof, or a prodrug thereof: 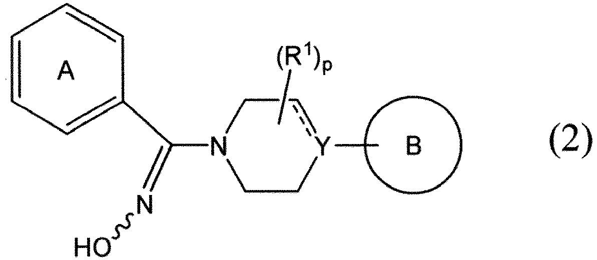 wherein
A is an optionally substituted benzene ring;
B is an optionally substituted aryl or an optionally substituted heteroaryl;
Y is a nitrogen atom or a carbon atom;
------ of -----Y is a single or double bond when Y is a carbon atom, or
------ of -----Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

### Advantageous Effects of Invention

A compound represented by formula (1), a compound represented by formula (2), a salt thereof, or a prodrug thereof has an action of preventing and/or treating acute kidney injury, e.g., kidney injury caused by administration of a platinum-containing drug, and has an action of activating autophagy in cells. The pharmaceutical composition and autophagy activator according to the present invention are distinctive, showing such actions even when administered orally.

### Brief Description of Drawings

Fig. 1 is microscopic images (400x) of section samples of renal cortex stained with PAS in Test Example 1. The upper-left image is of a vehicle administration group, the lower-left image is of a test compound administration group, the upper-right image is of a cisplatin administration group, and the lower-right image is of a cisplatin and test compound administration group.
Fig. 2 is a graph showing scores for renal tubular injury (PAS injury scores) obtained in Test Example 1.
Fig. 3 is graphs showing creatinine concentrations and urea nitrogen (BUN) concentrations obtained in Test Example 1. In Fig. 3, Cre represents a creatinine concentration, and BUN represents a urea nitrogen concentration.
Fig. 4 is microscopic images (200x) of section samples of renal cortex stained with p53 obtained in Test Example 2. The upper-left image is of a vehicle administration group, the lower-left image is of a test compound administration group, the upper-right image is of a cisplatin administration group, and the lower-right image is of a cisplatin and test compound administration group. The arrows in Fig. 4 indicate p53-positive cells.
Fig. 5 is a graph showing the number of p53-positive cells (average values) obtained in Test Example 2.
Fig. 6 is microscopic images (400x) of section samples of renal cortex stained with p62 obtained in Test Example 2. The upper-left image is of a vehicle administration group, the lower-left image is of a test compound administration group, the upper-right image is of a cisplatin administration group, and the lower-right image is of a cisplatin and test compound administration group. In Fig. 6, p62 positive dots are enclosed in circles.
Fig. 7 is a graph showing the number of p62 positive dots (average values) obtained in Test Example 2.
Fig. 8 is microscopic images (2400x) of section samples of renal cortex of GFP-LC3 transgenic mice obtained in Test Example 3. The upper-left image is of a control group (only cisplatin is administered), the lower-left image is of a chloroquine administration group, the upper-right image is of a test compound administration group, and the lower-right image is of a chloroquine and test compound administration group.
Fig. 9 is a graph showing the number of GFP-positive dots obtained in Test Example 3.
Fig. 10 shows the results of electrophoresis obtained in Test Example 4.
Fig. 11 is a graph showing the LC3-II protein level obtained in Test Example 4.
Fig. 12 is a graph showing the difference obtained by subtracting the LC3-II protein level without treatment with Bafilomycin A1 from the LC3-II protein level with treatment with Bafilomycin A1 obtained in Test Example 4.

### Description of Embodiments

One embodiment of the present invention is a pharmaceutical composition for preventing and/or treating acute kidney injury, comprising a compound represented by the following formula (1), a salt thereof, or a prodrug thereof.
Formula (1) 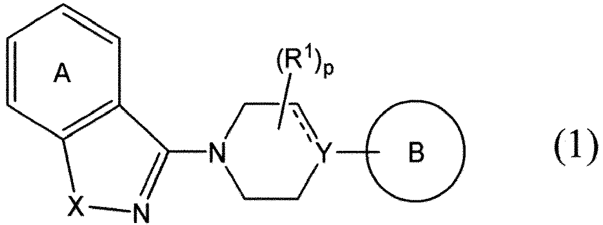 (wherein A is an optionally substituted benzene ring;
B is an optionally substituted aryl or an optionally substituted heteroaryl;
X is an oxygen atom or a sulfur atom;
Y is a nitrogen atom or a carbon atom;
------ of -----Y is a single or double bond when Y is a carbon atom, or
------ of -----Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo) .

In the present invention, examples of substituents for the "optionally substituted benzene ring" include halogen; hydroxyl; nitro; cyano; carboxyl; optionally substituted amino; optionally substituted cyclic amino; optionally substituted lower alkyl; optionally substituted lower alkoxy; lower alkoxycarbonyl, lower alkylsulfonyl; carbamoyl optionally substituted with lower alkyl or lower alkylsulfonyl; optionally substituted cyclic aminocarbonyl; sulfamoyl optionally substituted with lower alkyl; optionally substituted cyclic aminosulfonyl; tetrazolyl; and the like. Such substituents may be used singly or in a combination of two or more.

In the present invention, "aryl" may be, for example, a monocyclic or bicyclic aryl. Specific examples include phenyl, naphthyl, and the like.

In the present invention, the aryl in the "optionally substituted aryl" is as defined above. Examples of substituents for the optionally substituted aryl include halogen; hydroxyl; nitro; cyano; carboxyl; optionally substituted amino; optionally substituted cyclic amino; optionally substituted lower alkyl; optionally substituted lower alkoxy; lower alkoxycarbonyl; lower alkylsulfonyl; carbamoyl optionally substituted with lower alkyl or lower alkylsulfonyl; optionally substituted cyclic aminocarbonyl; sulfamoyl optionally substituted with lower alkyl; optionally substituted cyclic aminosulfonyl; tetrazolyl; oxo; and the like. Such substituents may be used singly or in a combination of two or more.

In the present invention, "oxo" is a group represented by "=O".

In the present invention, "heteroaryl" is, for example, a monocyclic or bicyclic nitrogen-containing heteroaryl. Specific examples include a monocyclic or bicyclic nitrogen-containing heteroaryl that contains one or more (for example, one to three, one or two, or one) nitrogen atoms on the ring and optionally further contains one or more (for example, one to three, one or two, or one) sulfur atoms or oxygen atoms as other heteroatoms. Specific examples of the heteroaryl include pyrrolyl, imidazolyl, triazolyl, tetrazolyl, pyrazolyl, pyridyl, pyrazinyl, pyrimidinyl, pyridadinyl, furyl, thienyl, oxazolyl, isooxazolyl, thiazolyl, isothiazolyl, furazanyl, oxadiazolyl, thiadiazolyl, indolyl, isoindolyl, benzofuranyl, isobenzofuranyl, benzothienyl, isobenzothienyl, indazolyl, quinolyl, isoquinolyl, purinyl, phthalazinyl, pteridyl, naphthyridinyl, quinoxalinyl, quinazolinyl, cinnolinyl, benzoxazolyl, benzothiazolyl, benzimidazolyl, benzofurazanyl, benzothiadiazolyl, benzotriazolyl, isoxazolo[4,5-d]pyridazyl, benzisoxazolyl, benzisothiazolyl, and the like. Preferred is a monocyclic nitrogen-containing heteroaryl or benzimidazolyl.

In the present invention, the heteroaryl in the "optionally substituted heteroaryl" is as defined above. Examples of substituents for the optionally substituted heteroaryl include halogen; hydroxyl; nitro; cyano; carboxyl; optionally substituted amino; optionally substituted cyclic amino; optionally substituted lower alkyl; optionally substituted lower alkoxy; lower alkoxycarbonyl; lower alkylsulfonyl; carbamoyl optionally substituted with lower alkyl or lower alkylsulfonyl; optionally substituted cyclic aminocarbonyl; sulfamoyl optionally substituted with lower alkyl; optionally substituted cyclic aminosulfonyl; tetrazolyl; oxo; and the like. Such substituents may be used singly or in a combination of two or more.

In the present invention, "lower alkyl" includes, for example, C₁-C₈ alkyl, preferably C₁-C₆ alkyl, more preferably C₁-C₄ alkyl, and particularly preferably C₁-C₃ alkyl, each containing a linear, branched, or cyclic structure. Specific examples of linear or branched lower alkyl include methyl, ethyl, 1-propyl, 2-propyl, 1-butyl, 2-butyl, isobutyl, t-butyl, n-pentyl, neopentyl, n-hexyl, isohexyl, 3-methylpentyl, and the like. Examples of lower alkyl containing a cyclic structure include cyclopropyl, cyclopropylmethyl, cyclobutyl, cyclobutylmethyl, cyclopentyl, cyclopentylmethyl, cyclohexyl, cyclohexylmethyl, cyclohexylethyl, and the like. Preferable examples include methyl, ethyl, 2-propyl, t-butyl, cyclopropyl, and the like.

In the present invention, examples of "halogen" include a fluorine atom, a chlorine atom, a bromine atom, an iodine atom, and the like. Preferable examples include a fluorine atom and a chlorine atom.

In the present invention, "optionally substituted amino" refers to an optionally substituted acyclic amino group. Examples of substituents for the amino group include lower alkyl (e.g., methyl, ethyl, and propyl), C₁-C₈ acyl (e.g., acetyl and propionyl), aryl (e.g., phenyl), and heteroaryl. Such substituents may be used singly or in a combination of two or more. Preferable examples of the optionally substituted amino include amino, methylamino, dimethylamino, ethylamino, diethylamino, cyclohexylamino, acetylamino, benzoylamino, phenylamino, and the like.

In the present invention, "cyclic amino" may be, for example, a 5- to 7-membered cyclic amino group containing a nitrogen atom as a ring-constituting atom and optionally further containing one or more (for example, one to three, one or two, or one) oxygen atoms. Specific examples include pyrrolidino, piperidino, piperazino, morpholino, and the like. Preferable examples include pyrrolidino, morpholino, and the like.

In the present invention, the cyclic amino in the "optionally substituted cyclic amino" is as defined above. Examples of substituents for the cyclic amino include lower alkyl, lower alkoxy, amino, hydroxyl, nitro, cyano, carboxyl, oxo, and the like. The cyclic amino may be substituted with at least one substituent selected from the group consisting of the substituents mentioned above. The number of substituents is, for example, 0, 1, 2, and 3, and preferably 0, 1, and 2. Specific examples of the optionally substituted cyclic amino include pyrrolidino, piperidino, piperazino, 4-methylpiperidino, morpholino, 2-pyrrolidonyl, and the like. Preferable examples include pyrrolidino, morpholino, and the like.

In the present invention, the lower alkyl in the "optionally substituted lower alkyl" is as defined above. Examples of substituents for the lower alkyl include hydroxyl; amino; C₁-C₈ alkylamino (e.g., methylamino, ethylamino, propylamino, and t-butylamino); C₁-C₈ alkoxy (e.g., methoxy, ethoxy, 1-propyloxy, 2-propyloxy, and t-butyloxy); halogen (e.g., a fluorine atom, a chlorine atom, and a bromine atom); halo-C₁-C₈ alkoxy (e.g., trifluoromethoxy); aliphatic heterocyclic groups (e.g., morpholino, piperidinyl, pyrrolidinyl, and 4-methyl-1-piperazino); aryl (e.g., phenyl and 1-naphthyl); heteroaryl (e.g., pyridyl, thienyl, and furanyl); carboxyl; C₁-C₈ alkoxycarbonyl (e.g., methoxycarbonyl, ethoxycarbonyl, 1-propoxycarbonyl, 2-propoxycarbonyl, and t-butoxycarbonyl); carbamoyl optionally substituted with lower alkyl (e.g., carbamoyl, methylaminocarbonyl, dimethylaminocarbonyl, and diethylaminocarbonyl); cyclic aminocarbonyl (e.g., pyrrolidinocarbonyl, piperidinocarbonyl, and morpholinocarbonyl); and the like. Examples of preferable substituents include methylamino, ethylamino, dimethylamino, diethylamino, methoxy, ethoxy, 2-propyloxy, t-butoxycarbonyl, hydroxyl, a fluorine atom, a chlorine atom, trichloromethyl, trifluoromethyl, trifluoromethoxy, morpholino, piperidino, pyrrolidino, carboxyl, methoxycarbonyl, ethoxycarbonyl, morpholinocarbonyl, phenyl, pyridyl, and the like. The optionally substituted lower alkyl may be substituted with at least one substituent selected from the group consisting of the substituents mentioned above. The number of substituents may be, for example, 0, 1, 2, or 3, and preferably 0, 1, or 2.

In the present invention, "halogen-substituted lower alkyl" means that all the hydrogen atoms in the alkyl are replaced by halogens. The halogens and lower alkyl in the halogen-substituted lower alkyl are as defined above. The halogens with which the alkyl is substituted are preferably the same. The halogen-substituted lower alkyl is preferably trichloromethyl or trifluoromethyl, and more preferably trifluoromethyl.

In the present invention, examples of "lower alkoxy" include C₁-C₈ alkoxy, preferably C₁-C₆ alkoxy, more preferably C₁-C₄ alkoxy, and particularly preferably C₁-C₃ alkoxy, each containing a linear, branched, or cyclic structure. Specific examples of linear or branched alkoxy include methoxy, ethoxy, 1-propoxy, 2-propoxy, 1-butoxy, 2-butoxy, isobutoxy, t-butoxy, n-pentyloxy, neopentyloxy, n-hexyloxy, isohexyloxy, 3-methylpentyloxy, and the like. Examples of alkoxy containing a cyclic structure include cyclopropoxy, cyclopropylmethoxy, cyclobutyloxy, cyclobutylmethoxy, cyclopentyloxy, cyclopentylmethoxy, cyclohexyloxy, cyclohexylmethoxy, cyclohexylethoxy, and the like. Preferable examples include methoxy, ethoxy, 2-propoxy, t-butoxy, cyclopropoxy, and the like.

In the present invention, the lower alkoxy in the "optionally substituted lower alkoxy" is as defined above. Examples of substituents for the lower alkoxy include hydroxyl; amino; C₁-C₈ alkylamino (e.g., methylamino, ethylamino, propylamino, and t-butylamino); C₁-C₈ alkoxy (e.g., methoxy, ethoxy, 1-propyloxy, 2-propyloxy, and t-butoxy); halogen (e.g., a fluorine atom, a chlorine atom, and a bromine atom); halo-C₁-C₈ alkoxy (e.g., trifluoromethoxy); aliphatic heterocyclic groups (e.g., morpholino, piperidinyl, pyrrolidinyl, and 4-methyl-1-piperazino); aryl (e.g., phenyl and 1-naphthyl); heteroaryl (e.g., pyridyl, thienyl, and furanyl); carboxyl; C₁-C₈ alkoxycarbonyl (e.g., methoxycarbonyl, ethoxycarbonyl, 1-propyloxycarbonyl, 2-propyloxycarbonyl, and t-butoxycarbonyl); carbamoyl optionally substituted with lower alkyl (e.g., carbamoyl, methylaminocarbonyl, dimethylaminocarbonyl, and diethylaminocarbonyl); cyclic aminocarbonyl (e.g., pyrrolidinocarbonyl, piperidinocarbonyl, and morpholinocarbonyl); and the like. Examples of preferable substituents include methylamino, ethylamino, dimethylamino, diethylamino, methoxy, ethoxy, 2-propyloxy, t-butoxycarbonyl, hydroxyl, a fluorine atom, a chlorine atom, trifluoro, morpholino, piperidino, pyrrolidino, carboxyl, methoxycarbonyl, morpholinocarbonyl, phenyl, pyridyl, and the like. The optionally substituted lower alkoxy may be substituted with at least one substituent selected from the group consisting of the substituents mentioned above. The number of substituents may be, for example, 0, 1, 2, or 3, and preferably 0, 1, or 2.

In the present invention, the lower alkoxy in the "lower alkoxycarbonyl" is as defined above. The lower alkoxycarbonyl is a group in which lower alkoxy, such as those mentioned above, is bound to carbonyl. Examples of the lower alkoxycarbonyl include C₁-C₈ alkoxycarbonyl containing a linear, branched, or cyclic structure. Specific examples of the linear or branched alkoxycarbonyl include methoxycarbonyl, ethoxycarbonyl, 1-propoxycarbonyl, 2-propoxycarbonyl, 1-butoxycarbonyl, 2-butoxycarbonyl, isobutoxycarbonyl, t-butoxycarbonyl, and the like. Examples of the C₁-C₈ alkoxycarbonyl containing a cyclic structure include cyclopropoxycarbonyl, cyclopropylmethoxycarbonyl, cyclobutyloxycarbonyl, cyclobutylmethoxycarbonyl, cyclopentyloxycarbonyl, cyclopentylmethoxycarbonyl, cyclohexyloxycarbonyl, cyclohexylmethoxycarbonyl, cyclohexylethoxycarbonyl, and the like. Preferable examples of the lower alkoxycarbonyl include methoxycarbonyl, ethoxycarbonyl, 2-propoxycarbonyl, cyclopropoxycarbonyl, and the like.

In the present invention, the lower alkyl in the "lower alkylsulfonyl" is as defined above. The lower alkylsulfonyl is a group in which lower alkyl, such as those mentioned above, is bound to sulfonyl. Examples of the lower alkylsulfonyl include C₁-C₈ alkylsulfonyl containing a linear, branched, or cyclic structure. Specific examples of the linear or branched alkylsulfonyl include methanesulfonyl, ethanesulfonyl, 1-propylsulfonyl, 2-propylsulfonyl, 1-butylsulfonyl, 2-butylsulfonyl, isobutylsulfonyl, t-butylsulfonyl, and the like. Examples of the C₁-C₈ alkylsulfonyl containing a cyclic structure include cyclopropylsulfonyl, cyclopropylmethylsulfonyl, cyclobutylsulfonyl, cyclobutylmethylsulfonyl, cyclopentylsulfonyl, cyclopentylmethylsulfonyl, cyclohexylsulfonyl, cyclohexylmethylsulfonyl, cyclohexylethylsulfonyl, and the like. Preferable examples include methanesulfonyl, ethanesulfonyl, 2-propanesulfonyl, cyclopropanesulfonyl, and the like.

In the present invention, the lower alkyl and lower alkylsulfonyl in the "carbamoyl optionally substituted with lower alkyl or lower alkylsulfonyl" are as defined above. The carbamoyl optionally substituted with lower alkyl or lower alkylsulfonyl includes "carbamoyl optionally substituted with lower alkyl" and "carbamoyl optionally substituted with lower alkylsulfonyl."

The "carbamoyl optionally substituted with lower alkyl" is a group in which one or two lower alkyl groups, such as those mentioned above, may be bound to carbamoyl. When two lower alkyl groups are bound, the lower alkyl groups may be the same or different. Examples of the carbamoyl optionally substituted with lower alkyl include carbamoyl; aminocarbonyl substituted with C₁-C₈ alkyl containing a linear, branched, or cyclic structure; and the like. Specific examples of the carbamoyl optionally substituted with lower alkyl include carbamoyl, methylaminocarbonyl, ethylaminocarbonyl, propylaminocarbonyl, 2-propylaminocarbonyl, dimethylaminocarbonyl, diethylaminocarbonyl, ethylmethylaminocarbonyl, methylpropylaminocarbonyl, dicyclohexylaminocarbonyl, and the like.

The "carbamoyl optionally substituted with lower alkylsulfonyl" is a group in which one or two lower alkylsulfonyl groups, such as those mentioned above, may be bound to carbamoyl. When two lower alkylsulfonyl groups are bound, the lower alkylsulfonyl groups may be the same or different. Examples of the carbamoyl optionally substituted with lower alkylsulfonyl include carbamoyl; aminocarbonyl substituted with C₁-C₈ alkylsulfonyl containing a linear, branched, or cyclic structure; and the like. Examples of the linear or branched C₁-C₈ alkylsulfonylaminocarbonyl include methanesulfonylaminocarbonyl, ethanesulfonylaminocarbonyl, 1-propylsulfonylaminocarbonyl, 2-propylsulfonylaminocarbonyl, 1-butylsulfonylaminocarbonyl, 2-butylsulfonylaminocarbonyl, isobutylsulfonylaminocarbonyl, t-butylsulfonylaminocarbonyl, and the like. Examples of the C₁-C₈ alkylsulfonylaminocarbonyl containing a cyclic structure include cyclopropylsulfonylaminocarbonyl, cyclopropylmethylsulfonylaminocarbonyl, cyclobutylsulfonylaminocarbonyl, cyclobutylmethylsulfonylaminocarbonyl, cyclopentylsulfonylaminocarbonyl, cyclopentylmethylsulfonylaminocarbonyl, cyclohexylsulfonylaminocarbonyl, cyclohexylmethylsulfonylaminocarbonyl, cyclohexylethylsulfonylaminocarbonyl, and the like. Preferable examples of the carbamoyl optionally substituted with lower alkylsulfonyl include carbamoyl, methanesulfonylaminocarbonyl, ethanesulfonylaminocarbonyl, 2-propylsulfonylaminocarbonyl, cyclopropylsulfonylaminocarbonyl, and the like.

In the present invention, the optionally substituted cyclic amino in the "optionally substituted cyclic aminocarbonyl" is as defined above. The optionally substituted cyclic aminocarbonyl is a group in which optionally substituted cyclic amino, such as those mentioned above, is bound to carbonyl. Specific examples of the optionally substituted cyclic aminocarbonyl include pyrrolidinocarbonyl, piperidinocarbonyl, piperazinocarbonyl, 4-methylpiperidino, morpholinocarbonyl, 2-pyrrolidonylcarbonyl, and the like. Preferable examples are pyrrolidinocarbonyl, morpholinocarbonyl, and the like.

In the present invention, the lower alkyl in the "sulfamoyl optionally substituted with lower alkyl" is as defined above. The sulfamoyl optionally substituted with lower alkyl is a group in which one or two lower alkyl groups, such as those mentioned above, may be bound to sulfamoyl. When two lower alkyl groups are bound, the lower alkyl groups may be the same or different. Examples of the sulfamoyl optionally substituted with lower alkyl include sulfamoyl; aminosulfonyl substituted with C₁-C₈ alkyl containing a linear, branched, or cyclic structure; and the like. Specific examples include sulfamoyl, methylaminosulfonyl, ethylaminosulfonyl, propylaminosulfonyl, 2-propylaminosulfonyl, dimethylaminophonyl, diethylaminosulfonyl, ethylmethylaminosulfonyl, methylpropylaminosulfonyl, dicyclohexylaminosulfonyl, and the like.

In the present invention, the optionally substituted cyclic amino in the "optionally substituted cyclic aminosulfonyl" is as defined above. The optionally substituted cyclic aminosulfonyl is a group in which optionally substituted cyclic amino, such as those mentioned above, is bound to sulfonyl. Specific examples of the optionally substituted cyclic aminosulfonyl include pyrrolidinosulfonyl, piperidinosulfonyl, piperazinosulfonyl, 4-methylpiperidinosulfonyl, morpholinosulfonyl, 4-piperidonylsulfonyl, and the like. Preferable examples are pyrrolidinosulfonyl, morpholinosulfonyl, and the like.

In the present invention, when Y is a carbon atom, ------ is a single or double bond. When Y is a nitrogen atom, ------ is a single bond.

In the compound represented by formula (1), A is an optionally substituted benzene ring. Examples of the substituent for A include at least one member selected from the group consisting of the following A-1 to A-16. When two or more substituents are present, the substituents may be the same or different from each other.
A-1) halogen,
A-2) hydroxyl,
A-3) nitro,
A-4) cyano,
A-5) carboxyl,
A-6) optionally substituted amino,
A-7) optionally substituted cyclic amino,
A-8) optionally substituted lower alkyl,
A-9) optionally substituted lower alkoxy,
A-10) lower alkoxycarbonyl,
A-11) lower alkylsulfonyl,
A-12) carbamoyl optionally substituted with lower alkyl or lower alkylsulfonyl,
A-13) optionally substituted cyclic aminocarbonyl,
A-14) sulfamoyl optionally substituted with lower alkyl,
A-15) optionally substituted cyclic aminosulfonyl, and
A-16) tetrazolyl.

The number of substituents for A is, for example, 0 to 5, 0 to 4, 0 to 3, preferably 0, 1, or 2, and more preferably 0 or 1. When two or more substituents are present, the substituents may be the same or different from each other.

Other examples of substituents for A include at least one member selected from the group consisting of A-1 and A-3 to A-16 described above; at least one member selected from the group consisting of the above A-1 and A-3 to A-16 excluding methoxy; and the like.

The substituent for A is preferably at least one member selected from the group consisting of halogen; lower alkoxy; carbamoyl optionally substituted with lower alkyl or lower alkylsulfonyl; and optionally halogen-substituted lower alkyl, more preferably at least one member selected from the group consisting of halogen; lower alkoxy; carbamoyl; and optionally halogen-substituted lower alkyl, even more preferably at least one member selected from the group consisting of halogen, methoxy, ethoxy, carbamoyl, fluoromethyl, difluoromethyl, trifluoromethyl, fluoroethyl, difluoroethyl, and trifluoroethyl, still more preferably at least one member selected from the group consisting of halogen, methoxy, ethoxy, carbamoyl, fluoromethyl, difluoromethyl, trifluoromethyl, fluoroethyl, difluoroethyl, and trifluoroethyl, and particularly preferably at least one member selected from the group consisting of a chlorine atom, a fluorine atom, and trifluoromethyl.

In the compound represented by formula (1), the substituent for A may be bound to any of the carbon atoms at the 4-position, 5-position, 6-position, and 7-position of the benzisoxazole or benzisothiazole skeleton. Preferably, the substituent is bound to at least one of the carbon atoms at the 4-position, 5-position, and 6-position, more preferably to carbon atoms at the 4-position and/or 5-position, and particularly preferably to the carbon atom at the 4-position. In the present invention, the position numbers of the atoms constituting the benzisoxazole or benzisothiazole skeleton are as follows. 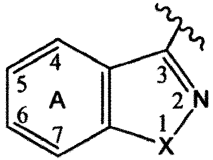 (wherein A and X are as defined above).

In the compound represented by formula (1), particularly preferable A is a benzene ring wherein halogen, lower alkoxy, or optionally halogen-substituted lower alkyl is bound to the carbon atom at the 4-position of the benzisoxazole or benzisothiazole skeleton, and carbon atoms at the 5-, 6-, and 7-positions are not substituted.

In the compound represented by formula (1), B is an optionally substituted aryl or an optionally substituted heteroaryl. The optionally substituted aryl or optionally substituted heteroaryl is as defined above. The aryl is, for example, phenyl or naphthyl, and is preferably phenyl. The heteroaryl is preferably a monocyclic nitrogen-containing heteroaryl that does not contain any other heteroatom as a ring-constituting atom, or benzimidazolyl. The monocyclic nitrogen-containing heteroaryl that does not contain any other heteroatom as a ring-constituting atom is preferably a 5- or 6-membered heteroaryl containing one nitrogen atom as a ring-constituting heteroatom. Examples include pyrrolyl and pyridyl, preferably pyridyl, and more preferably 2-pyridyl. The benzimidazolyl is preferably benzimidazol-3-yl.

When B is a monocyclic aryl, B may be substituted with at least one substituent selected from the group consisting of the following B-1 to B-16. When B is a monocyclic or bicyclic heteroaryl, B may be substituted with at least one substituent selected from the group consisting of the following B-1 to B-17:
B-1) halogen,
B-2) hydroxyl,
B-3) nitro,
B-4) cyano,
B-5) carboxyl,
B-6) optionally substituted amino,
B-7) optionally substituted cyclic amino,
B-8) optionally substituted lower alkyl,
B-9) optionally substituted lower alkoxy,
B-10) lower alkoxycarbonyl,
B-11) lower alkylsulfonyl,
B-12) carbamoyl optionally substituted with lower alkyl or lower alkylsulfonyl,
B-13) optionally substituted cyclic aminocarbonyl,
B-14) sulfamoyl optionally substituted with lower alkyl,
B-15) optionally substituted cyclic aminosulfonyl,
B-16) tetrazolyl, and
B-17) oxo.

The number of substituents for B is, for example, 0 or at least 1, 0 to 5, 0 to 4, preferably 0 to 3, and more preferably 0, 1, or 2. When two or more substituents are present, the substituents may be the same or different from each other.

The substituent for B is, for example, at least one member selected from the group consisting of halogen; carboxyl; optionally substituted lower alkyl; lower alkoxycarbonyl; carbamoyl optionally substituted with lower alkyl or lower alkylsulfonyl; and optionally substituted cyclic aminocarbonyl. Specific examples include at least one member selected from the group consisting of halogen, carboxyl, methyl, ethyl, 1-propyl, 2-propyl, hydroxymethyl, carboxymethyl, trichloromethyl, trifluoromethyl, methoxycarbonyl, ethoxycarbonyl, t-butoxycarbonyl, methylaminocarbonyl, dimethylaminocarbonyl, ethylmethylaminocarbonyl, methanesulfonylaminocarbonyl, pyrrolidinocarbonyl, and morpholinocarbonyl.

The substituent for B is preferably, for example, at least one member selected from the group consisting of halogen; carboxyl; lower alkyl; halogen-substituted lower alkyl; lower alkoxycarbonyl; and carbamoyl optionally substituted with lower alkyl. Specific examples include at least one member selected from the group consisting of halogen, carboxyl, methyl, ethyl, trichloromethyl, trifluoromethyl, methoxycarbonyl, ethoxycarbonyl, n-propoxycarbonyl, isopropoxycarbonyl, monomethylaminocarbonyl, and dimethylaminocarbonyl.

Particularly preferable examples of substituents for B include at least one member selected from the group consisting of a chlorine atom, a fluorine atom, methyl, carboxyl, methoxycarbonyl, ethoxycarbonyl, methylaminocarbonyl, and dimethylaminocarbonyl.

In the compound represented by formula (1), when Y is a nitrogen atom, B is preferably an optionally substituted phenyl or an optionally substituted pyridyl. When Y is a carbon atom, B is preferably an optionally substituted phenyl, an optionally substituted pyridyl, or 2-oxobenzoimidazole-3-yl, and more preferably an optionally substituted phenyl or an optionally substituted pyridyl.

In the compound represented by formula (1), when B is substituted pyridyl or substituted phenyl, one or two carbon atoms, preferably one carbon atom, in the ortho-positions with respect to the Y-bound carbon atom on the pyridine or benzene ring are preferably substituted. The substituent(s) bound to the carbon atom(s) in the ortho-position(s) may be any substituent for B. The substituent is preferably halogen, more preferably a chlorine atom or a fluorine atom, and even more preferably a chlorine atom.

In the compound represented by formula (1), when B is substituted pyridyl or substituted phenyl, the carbon atom in the para-position with respect to the Y-bound carbon atom on the pyridine or benzene ring is preferably unsubstituted or substituted with carboxyl.

Further, in the compound represented by formula (1), when B is substituted pyridyl or substituted phenyl, all the carbon atoms in the meta-positions with respect to the Y-bound carbon atom on the pyridine or benzene ring are preferably unsubstituted.

In the compound represented by formula (1), when B is substituted pyridyl or substituted phenyl, it is more preferable that one or two carbon atoms, more preferably one carbon atom, in the ortho-positions with respect to the Y-bound carbon atom on the pyridine or benzene ring are substituted with a chlorine atom or a fluorine atom, that carbon atoms in the meta-positions are unsubstituted, and that the carbon atom in the para-position is unsubstituted or substituted with carboxyl, methoxycarbonyl, or ethoxycarbonyl.

When Y is a nitrogen atom and B is substituted 2-pyridyl, it is particularly preferable that one of the carbon atoms in the ortho-positions with respect to the Y-bound carbon atom on the pyridine ring is substituted with a chlorine atom or a fluorine atom, that all the carbon atoms in the meta-positions are unsubstituted, and that the carbon atom in the para-position is unsubstituted or substituted with carboxyl.

When Y is a nitrogen atom and B is substituted phenyl, it is particularly preferable that one of the two carbon atoms in the ortho-positions with respect to the Y-bound carbon atom on the benzene ring is substituted with a chlorine atom or a fluorine atom, and that all the other carbon atoms that constitute the phenyl are unsubstituted.

When Y is a carbon atom and B is substituted 2-pyridyl, it is particularly preferable that one of the carbon atoms in the ortho-positions with respect to the Y-bound carbon atom on the pyridine ring is substituted with a chlorine atom or a fluorine atom, that all the carbon atoms in the meta-positions are unsubstituted, and that the carbon atom in the para-position is unsubstituted or substituted with carboxyl, methoxycarbonyl, or ethoxycarbonyl.

When Y is a carbon atom and B is substituted phenyl, it is particularly preferable that one of the two carbon atoms in the ortho-positions with respect to the Y-bound carbon atom on the benzene ring is substituted with a chlorine atom or a fluorine atom, that the other one of the carbon atoms in the ortho-positions is unsubstituted, that all the carbon atoms in the meta-positions are unsubstituted, and that the carbon atom in the para-position is unsubstituted or substituted with carboxyl, methoxycarbonyl, or ethoxycarbonyl.

In the compound represented by formula (1), X is an oxygen atom or a sulfur atom, and is preferably an oxygen atom.

In the compound represented by formula (1), Y is a nitrogen atom or a carbon atom, and is preferably a nitrogen atom.

In the compound represented by formula (1), Y is a nitrogen atom or a carbon atom, and is preferably a nitrogen atom.

When Y is a carbon atom, ------ is a single or double bond. When Y is a nitrogen atom, ------ is a single bond.

In the compound represented by formula (1), each R¹ independently represents lower alkyl, or two R¹s may be bound to each other to form a spiro ring or a crosslinked structure, or two R¹s may be bound to each other to form a saturated fused heterocycle together with nitrogen and carbon atoms constituting a ring containing Y.

When R¹ is lower alkyl, preferable examples of R¹ include linear or branched C₁-C₃ alkyl. R¹ is more preferably methyl or ethyl, and even more preferably methyl.

When two R¹s are bound to each other to form a spiro ring or a crosslinked structure, forming a spiro ring means that two R¹s are both bound to one of the carbon atoms constituting the ring containing Y in formula (1) and the two R¹s are bound to each other to form a ring together with the carbon atom.

When two R¹s are bound to each other to form a spiro ring or a crosslinked structure, forming a crosslinked structure means that one R¹ group each is bound to two of the carbon atoms constituting the ring containing Y in formula (1) and the R¹s are bound to each other.

Examples of the case in which two R¹s are bound to each other to form a spiro ring or a crosslinked structure include a case in which two R¹s are bound to each other to form methylene, dimethylene, trimethylene, or tetramethylene, thus forming a crosslinked structure, and a case in which two R¹s are bound to each other to form dimethylene or trimethylene, thus forming a spiro ring. A preferable example is a case in which two R¹s are bound to each other to form methylene, dimethylene, or trimethylene, thus forming a crosslinked structure. A crosslinked structure formed by dimethylene, which is represented by the following structural formula, is particularly preferable. 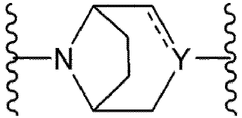 (wherein Y and ------ are as defined above.)

The phrase "two R¹s are bound to each other to form a saturated fused heterocycle together with nitrogen and carbon atoms constituting a ring containing Y" means that one R¹ each is bound to two adjacent carbon atoms among the carbon atoms that constitute a ring containing Y in formula (1) and the R¹s are bound to each other to form a saturated fused heterocycle together with nitrogen and carbon atoms constituting a ring containing Y. The saturated fused heterocycle referred to herein means a fused bicyclic ring of a heterocycle containing Y (a pyrazine ring or a piperidine ring) and a saturated carbon ring containing R¹.

Examples of the saturated fused heterocycle include a fused ring of a pyrazine ring or a piperidine ring and a cyclopentane ring or a cyclohexane ring. Specific examples of the saturated fused heterocycle include octahydrocyclopentapyridine, octahydrocyclopentapyrazine, decahydroquinoline, decahydroquinoxaline, and the like.

R¹ is preferably a crosslinked structure formed by C₁-C₃ alkyl or dimethylene, more preferably a crosslinked structure formed by methyl, ethyl, or dimethylene represented by the above structural formula.

In the compound represented by formula (1), p is 0, 1, or 2.

In the compound represented by formula (1), (R¹)ₚ may be oxo.

Among the compounds represented by formula (1), salts thereof, or prodrugs thereof, the compound represented by the following formula (1A), a salt thereof, or a prodrug thereof is preferable. A pharmaceutical composition for preventing and/or treating acute kidney injury, comprising a compound represented by the following formula (1A), a salt thereof, or a prodrug thereof is also included within the scope of the present invention. 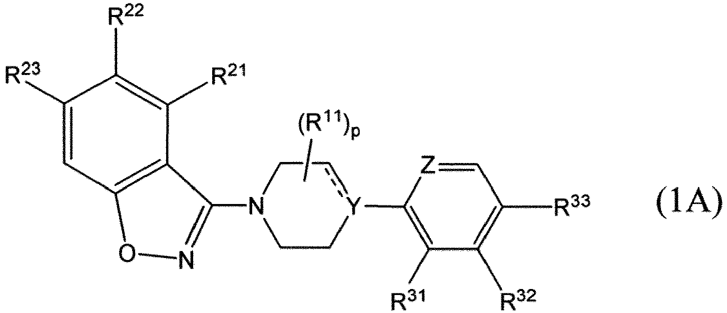 (wherein Z is a nitrogen atom or CH;
Y is a nitrogen atom or a carbon atom;
------ of -----Y is a single or double bond when Y is a carbon atom, or
------ of -----Y is a single bond when Y is a nitrogen atom; each R¹¹ independently represents methyl or ethyl, or
two R¹¹s may be bound to each other to form a crosslinked structure by methylene, dimethylene, or trimethylene;
p is 0, 1, or 2; or
(R¹¹)ₚ is oxo;
R²¹, R²², and R²³ independently represent a hydrogen atom, halogen, carbamoyl, or trifluoromethyl; and
R³¹, R³², and R³³ independently represent a hydrogen atom, halogen, halogen-substituted lower alkyl, methyl, carboxyl, lower alkoxycarbonyl, monomethylaminocarbonyl, or dimethylaminocarbonyl).

In the compound represented by formula (1A), Z is a nitrogen atom or CH. When Y is a nitrogen atom, Z is preferably a nitrogen atom.

In the compound represented by formula (1A), Y is a nitrogen atom or a carbon atom.

In the compound represented by formula (1A), each R¹¹ independently represents methyl or ethyl, or two R¹¹s may be bound to each other to form a crosslinked structure by methylene, dimethylene, or trimethylene.

R¹¹ is preferably methyl or ethyl, or a crosslinked structure formed by dimethylene or trimethylene, and more preferably methyl or a crosslinked structure formed by diethylene.

The case in which two R¹¹s are bound to each other to form a crosslinked structure by methylene, dimethylene, or trimethylene refers to a case in which one R¹¹ each is bound to two carbon atoms among the carbon atoms that constitute the ring containing Y in formula (1A) and the R¹¹s are bound to each other to form methylene, dimethylene, or trimethylene, thus forming a crosslinked structure on the piperazine ring.

When the ring containing Y in formula (1A) is substituted with R¹¹, (R¹¹)ₚ is preferably oxo, or the ring is preferably represented by the following structural formula: 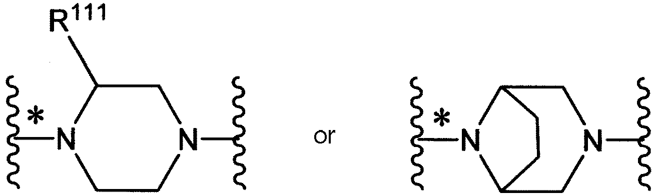 (wherein * represents the side bound to the carbon atom at the 3-position of isobenzoxazole, and R¹¹¹ represents C₁-C₃ alkyl) . R¹¹¹ is preferably methyl or ethyl, and more preferably methyl.

In formula (1A), (R¹¹)ₚ may be oxo.

In the compound represented by formula (1A), R²¹, R²², and R²³ independently represent a hydrogen atom, halogen, carbamoyl, or trifluoromethyl, and at least one of R²¹, R²², and R²³ is preferably halogen, carbamoyl, or trifluoromethyl. R²¹ is preferably a chlorine atom, a fluorine atom, carbamoyl, or trifluoromethyl, and more preferably a chlorine atom or trifluoromethyl. R²² is preferably a hydrogen atom, a chlorine atom, or trifluoromethyl, and more preferably a hydrogen atom. R²³ is preferably a hydrogen atom, a chlorine atom, or trifluoromethyl, and more preferably a hydrogen atom. It is particularly preferable that R²¹ is halogen (preferably a chlorine atom or a fluorine atom) or trifluoromethyl, and that R²² and R²³ are both a hydrogen atom.

In the compound represented by formula (1A), R³¹, R³², and R³³ independently represent a hydrogen atom, halogen, halogen-substituted lower alkyl, methyl, carboxyl, lower alkoxycarbonyl, monomethylaminocarbonyl, or dimethylaminocarbonyl. R³¹ is preferably a hydrogen atom, halogen, trichloromethyl, trifluoromethyl, or methyl, and more preferably halogen, trichloromethyl, trifluoromethyl, or methyl, and particularly preferably a chlorine atom. R³² is preferably a hydrogen atom, halogen, or methyl, and more preferably a hydrogen atom. R³³ is preferably a hydrogen atom, halogen, carboxyl, methoxycarbonyl, ethoxycarbonyl, monomethylaminocarbonyl, or dimethylaminocarbonyl, more preferably a hydrogen atom, carboxyl, methoxycarbonyl, or ethoxycarbonyl, and particularly preferably a hydrogen atom or carboxyl.

For R³¹, R³², and R³³, it is preferable that R³¹ is halogen (preferably a chlorine atom or a fluorine atom), R³² is a hydrogen atom, and R³³ is a hydrogen atom or carboxyl. When R²¹ is halogen (preferably a chlorine atom), it is preferable that R³¹ is halogen (preferably a chlorine atom or a fluorine atom), R³² is a hydrogen atom, and R³³ is a hydrogen atom. When R²¹ is trihalomethyl (preferably trifluoromethyl), it is preferable that R³¹ is halogen (preferably a chlorine atom or a fluorine atom), R³² is a hydrogen atom, and R³³ is a hydrogen atom, carboxyl, methoxycarbonyl, or ethoxycarbonyl. When R²¹ is carbamoyl, it is preferable that R³¹ is halogen (preferably a chlorine atom or a fluorine atom, more preferably a chlorine atom), R³² is a hydrogen atom, and R³³ is a hydrogen atom. It is also preferable that R²¹ is a chlorine atom or trifluoromethyl, R²² and R²³ are both a hydrogen atom, R³¹ is a chlorine atom, R³² is a hydrogen atom, and R³³ is a hydrogen atom or carboxyl.

Specific examples of the compound represented by formula (1), a salt thereof, or a prodrug thereof include Compound 011, Compound 021, Compound 031, Compound 041, Compound 051, Compound 061, Compound 071, Compound 081, Compound 091, Compound 101, Compound 111, Compound 121, Compound 131, Compound 141, Compound 151, Compound 161, Compound 171, Compound 181, Compound 191, Compound 201, Compound 211, Compound 221, Compound 231, Compound 241, Compound 251, Compound 261, Compound 271, Compound 281, Compound 291, Compound 301, Compound 311, Compound 321, Compound 331, Compound 341, Compound 351, Compound 361, Compound 371, Compound 381, Compound 391, Compound 401, Compound 411, Compound 421, and Compound 431. Preferable examples include the following compounds, salts thereof, and prodrugs thereof.

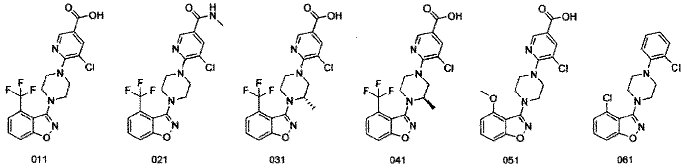

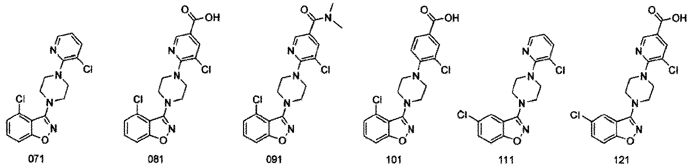

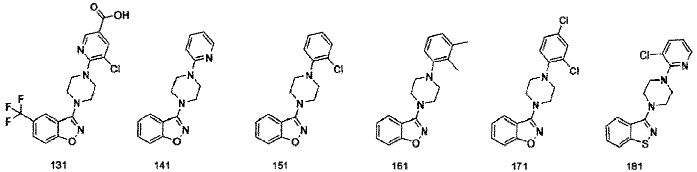

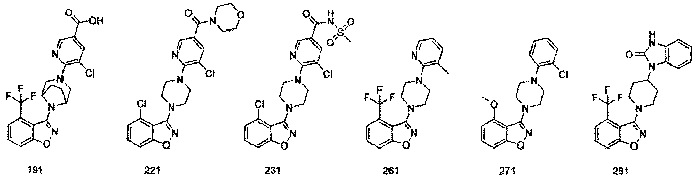

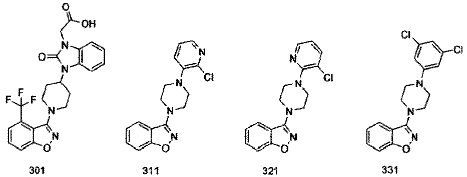

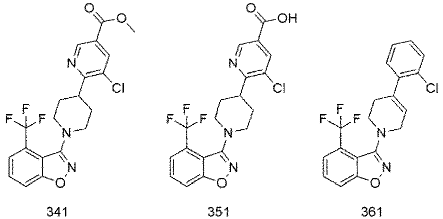

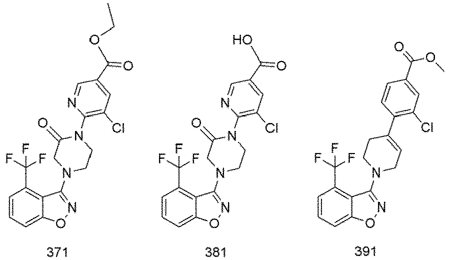

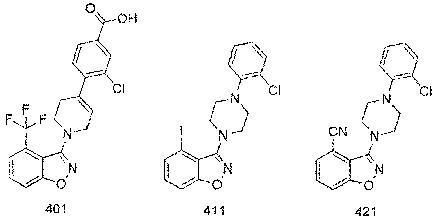

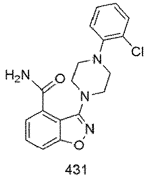

The compound represented by formula (1), a salt thereof, or a prodrug thereof is
more preferably Compound 011, Compound 021, Compound 031, Compound 041, Compound 061, Compound 071, Compound 081, Compound 091, Compound 101, Compound 111, Compound 121, Compound 131, Compound 141, Compound 151, Compound 161, Compound 171, Compound 191, Compound 221, Compound 281, Compound 311, Compound 321, Compound 331, Compound 341, Compound 351, Compound 361, Compound 371, Compound 381, Compound 391, Compound 401, or Compound 431; a salt thereof; or a prodrug thereof,
even more preferably Compound 011, Compound 021, Compound 031, Compound 041, Compound 061, Compound 071, Compound 081, Compound 091, Compound 101, Compound 111, Compound 121, Compound 131, Compound 141, Compound 151, Compound 161, Compound 171, Compound 191, Compound 321, Compound 351, Compound 361, Compound 371, Compound 381, Compound 401, or Compound 431; a salt thereof; or a prodrug thereof,
still more preferably Compound 011, Compound 031, Compound 041, Compound 061, Compound 071, Compound 191, Compound 361, Compound 371, Compound 381, Compound 401, or Compound 431; a salt thereof; or a prodrug thereof; and
particularly preferably Compound 011, Compound 031, Compound 041, Compound 061, Compound 071, Compound 191, Compound 361, Compound 371, Compound 381, or Compound 401; a salt thereof; or a prodrug thereof.

One embodiment of the present invention includes a pharmaceutical composition for preventing and/or treating acute kidney injury, comprising a compound represented by the following formula (2), a salt thereof, or a prodrug thereof. Since this compound is structurally similar to the compound represented by formula (1), the compound represented by the following formula (2), a salt thereof, or a prodrug thereof can have a preventive and/or therapeutic effect on acute kidney injury. Further, the compound, a salt thereof, or a prodrug thereof can activate autophagy. Furthermore, the compound or a salt thereof can also be used as an intermediate compound for the compound represented by formula (1). 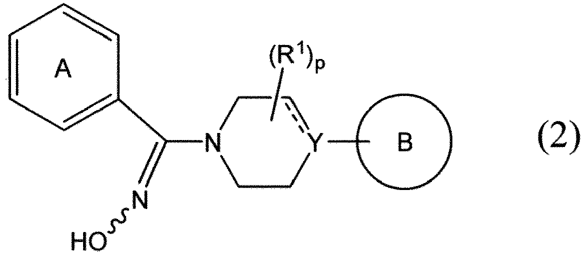 (wherein A, B, Y, R¹, p, and ------ are as defined above).

In the compound represented by formula (2), A, B, Y, R¹, p, and ------ are as defined above.

In formula (2), it is preferable that one or two of the carbon atoms in the ortho-positions with respect to the A-ring (benzene-ring) carbon atom that is bound to the carbon atom constituting the oxime structure are substituted. The substituents here may be the same as those for the A ring (benzene ring) in formula (1), unless otherwise stated. Thus, the substituents may be, for example, at least one substituent selected from the group consisting of A-1 to A-16 above.

Further, in formula (2), the bond between the nitrogen atom and the hydroxyl group is represented by a wavy line, which is a line indicated below. This line indicates that the compound represented by formula (2) may be an E-isomer, Z-isomer, or a blend thereof, wherein the E-isomer and Z-isomer are geometric isomers that are present due to the partial structure >C=N-OH of the compound. The same applies to compounds other than the compound of formula (2) that include the wavy line.

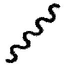

Among the compounds represented by formula (2), salts thereof, or prodrugs thereof, the compound represented by the following formula (2A), a salt thereof, or a prodrug thereof is preferable. 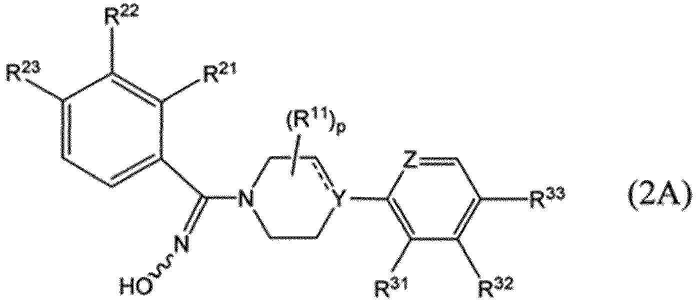 (wherein Z, Y, R¹¹, p, R²¹, R²², R²³, R³¹, R³², R³³, and ------ are as defined above.)

In the compound represented by formula (2A), Z, Y, R¹¹, p, R²¹, R²², R²³, R³¹, R³², R³³, and ------ are as defined above.

Among the compounds represented by formula (2), salts thereof, or prodrugs thereof, the compound represented by the following formula (2B), a salt thereof, or a prodrug thereof is preferable. The compound represented by formula (2B) or a salt thereof is also preferable as an intermediate compound for producing the compound represented by formula (1): 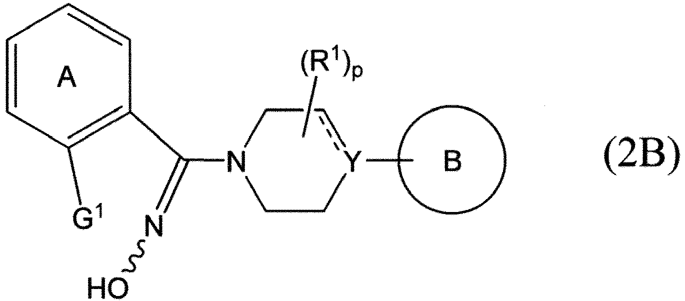 (wherein, A, B, Y, R¹, p, and ------ are as defined above, and G¹ is halogen, optionally halogen-substituted lower alkylsulfonyl, or benzenesulfonyl optionally substituted with lower alkyl or nitro).

In the compound represented by formula (2B), A, B, Y, R¹, p, and ------ are as defined above. The compound represented by formula (2B) or a salt thereof includes E-isomer and Z-isomer, which are geometric isomers that are present due to the partial structure >C=N-OH of the compound. When the compound represented by formula (2B) or a salt thereof is used as an intermediate compound for producing the compound represented by formula (1), the E-isomer is preferable.

Examples of the halogen represented by G¹ include a chlorine atom, a fluorine atom, a bromine atom, and an iodine atom.

The lower alkylsulfonyl in the optionally halogen-substituted lower alkylsulfonyl represented by G¹ is as defined above. The lower alkylsulfonyl is a group in which lower alkyl is bound to sulfonyl. The lower alkyl may be substituted with halogen. Examples of the optionally halogen-substituted lower alkylsulfonyl include linear or branched C₁-C₆ alkyl (preferably C₁-C₄ alkyl, and more preferably C₁-C₃ alkyl) sulfonyl that may be substituted with one to three halogens. Specific examples include methanesulfonyl, ethanesulfonyl, trifluoromethanesulfonyl, and the like.

Examples of the benzenesulfonyl optionally substituted with lower alkyl represented by G¹ include benzenesulfonyl that may be substituted with one to three (preferably one or two, and more preferably one) linear or branched C₁-C₆ alkyl groups (preferably C₁-C₄ alkyl, and more preferably C₁-C₃ alkyl) groups. Specific examples include p-toluenesulfonyl and the like.

Examples of the benzenesulfonyl optionally substituted with nitro represented by G¹ include benzenesulfonyl that may be substituted with one to three (preferably one) nitro groups. Specific examples include o-nitrobenzenesulfonyl, p-nitrobenzenesulfonyl, and the like.

G¹ is preferably a chlorine atom, a fluorine atom, a bromine atom, methanesulfonyl, ethanesulfonyl, trifluoromethanesulfonyl, p-toluenesulfonyl, or p-nitrobenzenesulfonyl. G¹ is more preferably a chlorine atom or a bromine atom.

The compound represented by formula (2), a salt thereof, or a prodrug thereof includes, for example, the following compounds, salts thereof, or prodrugs thereof.

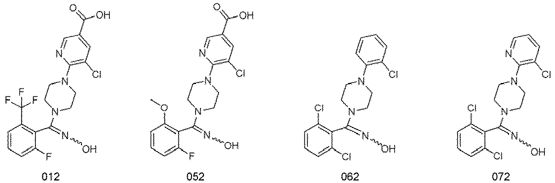

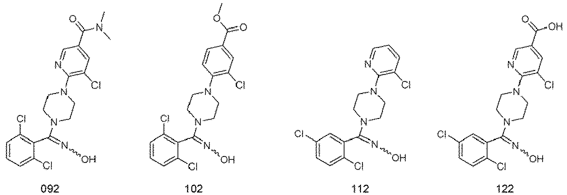

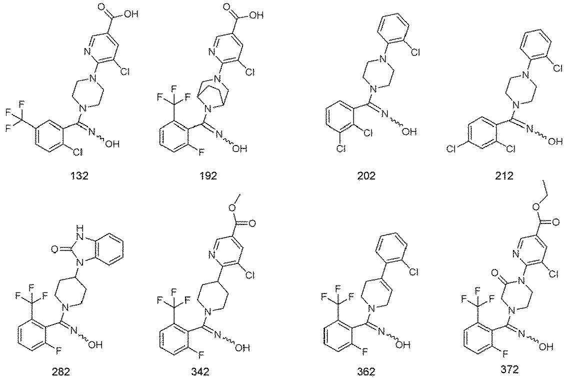

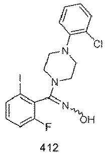

The compound represented by formula (2), a salt thereof, or a prodrug thereof is preferably Compound 062, Compound 202, Compound 362, or Compound 372; a salt thereof; or a prodrug thereof; and is more preferably (E)-isomer of Compound 202, (E)-isomer of Compound 362, (Z)-isomer of Compound 362, or (Z)-isomer of Compound 372; a salt thereof; or a prodrug thereof.

The compound described above can be produced, for example, by appropriate modification or a combination of any of the following: Production Methods 1 to 3 described below in detail, similar methods, known methods, and the like. The compounds to be used as starting material compounds may be used in the form of salts. The methods shown below are merely illustrative, and other methods can also be used as appropriate based on the knowledge of people skilled in organic synthesis. When 1,2-benzisothiazole or a derivative thereof or 1,2-benzisoxazole or a derivative thereof that is not a commercial product is used as a starting material compound, the compound can be produced and prepared with reference to the method disclosed in the following publication:
Advances in Heterocyclic Chemistry, Heterocyclic Chemistry in the 21st Century: A Tribute to Alan Katritzky, Elsevier, Cambridge (2017). R. A. Shastri, Review on Synthesis of 3-Substituted 1,2-Benzisoxazole Derivatives, Chem. Sci. Trans., 2016: 5; 8-20.

In each reaction for the production, functional groups can be protected as desired. With respect to the protecting groups and the technique of protection with protecting groups and deprotection, known methods, such as the method described in the following can be appropriately used: T. W. Greene and P. G. M. Wuts, Protective Groups in Organic Synthesis, 3rd Ed., John Wiley and Sons, Inc., New York (1999).

### Production Method 1

In an embodiment, the compound represented by formula (1) can be produced by the synthesis scheme illustrated in the following Reaction Formula-1. Specifically, the compound represented by formula (1) can be produced from the compound represented by formula (3) and the compound represented by formula (4). 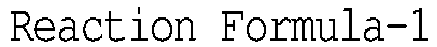 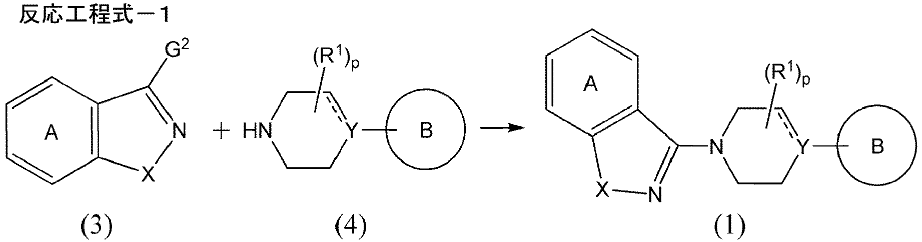 (wherein A, B, Y, R¹, p, and ------ are as defined above; and G² represents halogen, optionally halogen-substituted lower alkylsulfonyl, or benzenesulfonyl optionally substituted with lower alkyl or nitro.)

In Reaction Formula-1, A, B, Y, R¹, p, and ------ are as defined above.

Examples of halogen represented by G² include a chlorine atom, a fluorine atom, a bromine atom, and an iodine atom.

The "lower alkyl" in the optionally halogen-substituted lower alkylsulfonyl represented by G² is as defined above. The lower alkylsulfonyl is a group in which lower alkyl is bound to sulfonyl, and the lower alkyl may optionally be substituted with one or more halogens. Examples of the optionally halogen-substituted lower alkylsulfonyl include linear or branched C1-C6 alkyl (preferably C1-C4 alkyl, more preferably C1-C3 alkyl) sulfonyl that may be substituted with 1 to 3 halogens, specifically, methanesulfonyl, ethanesulfonyl, trifluoromethanesulfonyl, and the like.

Examples of the benzenesulfonyl optionally substituted with lower alkyl represented by G² include benzenesulfonyl that may be substituted with 1 to 3 linear or branched C1-C6 alkyl groups (preferably C1-C4 alkyl, more preferably C1-C3 alkyl), and specifically, p-toluenesulfonyl and the like.

Examples of the benzenesulfonyl optionally substituted with nitro represented by G² include benzenesulfonyl that may be substituted with 1 to 3 (preferably 1) nitro groups, specifically, o-nitrobenzenesulfonyl, p-nitrobenzenesulfonyl, and the like.

G² is preferably a chlorine atom, a fluorine atom, a bromine atom, methanesulfonyl, ethanesulfonyl, trifluoromethanesulfonyl, p-toluenesulfonyl, or p-nitrobenzenesulfonyl.

The reaction of the compound represented by formula (3) with the compound represented by formula (4) can be performed, for example, in an inert solvent in the presence or absence of a base. An activating reagent may optionally be further added to the reaction system. The compound represented by formula (3) and the compound represented by formula (4) are known compounds and can be produced by a known method.

Examples of inert solvents include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of bases include metal hydrides, such as sodium hydride and potassium hydride; metal hydroxides, such as potassium hydroxide and sodium hydroxide; metal carbonates, such as potassium carbonate, potassium hydrogen carbonate, sodium carbonate, sodium hydrogen carbonate, and cesium carbonate; alkyl amines, such as triethylamine and ethyldiisopropylamine; and metal alkoxides, such as sodium methoxide and potassium t-butoxide.

The amount of the base for use is typically 1 mol or more, preferably 1 to 5 mol, and more preferably 1 to 2 mol, per mol of the compound represented by formula (4).

The amount of the compound represented by formula (3) for use is typically 0.2 mol or more, preferably 0.2 to 2 mol, and more preferably 0.2 to 1.5 mol, per mol of the compound represented by formula (4).

The reaction temperature is typically -50°C to 180°C, preferably -30°C to 180°C, and more preferably -10°C to 180°C. Microwaves may be used for facilitating the reaction, and the reaction temperature in this case is, for example, 80°C to 180°C, and preferably 100°C to 180°C. The reaction time is typically 10 minutes to 48 hours, and preferably 10 minutes to 24 hours.

### Production Method 2

In an embodiment, the compound represented by formula (1B) can be produced by the synthesis scheme illustrated in the following Reaction Formula-2. Specifically, the compound represented by formula (1B) can be produced from the compound represented by formula (5) and the compound represented by formula (6). 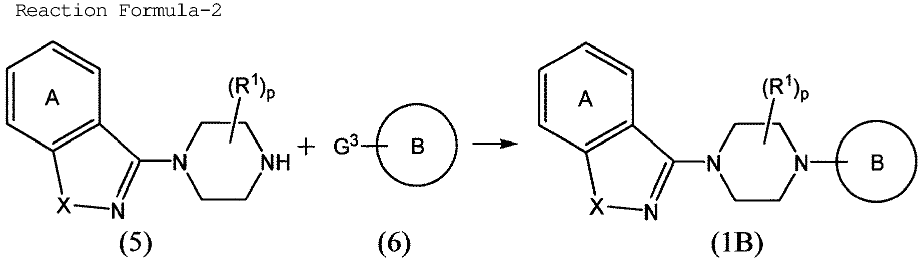 (wherein A, B, X, R¹, and p are as defined above, and G³ represents halogen, optionally halogen-substituted lower alkylsulfonyl, or benzenesulfonyl optionally substituted with lower alkyl or nitro.)

In Reaction Formula-2, A, B, X, R¹, and p are as defined above.

Examples of halogens represented by G³ include a chlorine atom, a fluorine atom, a bromine atom, and an iodine atom.

The "lower alkyl" in the optionally halogen-substituted lower alkylsulfonyl represented by G³ is as defined above. The lower alkylsulfonyl is a group in which lower alkyl is bound to sulfonyl, and the lower alkyl may optionally be substituted with one or more halogens. Examples of the optionally halogen-substituted lower alkylsulfonyl include linear or branched C1-C6 alkyl (preferably C1-C4 alkyl, more preferably C1-C3 alkyl) sulfonyl that may be substituted with 1 to 3 halogens, specifically, methanesulfonyl, ethanesulfonyl, trifluoromethanesulfonyl, and the like.

Examples of the benzenesulfonyl optionally substituted with lower alkyl represented by G³ include benzenesulfonyl that may be substituted with 1 to 3 linear or branched C1-C6 alkyl groups (preferably C1-C4 alkyl, more preferably C1-C3 alkyl), and specifically, p-toluenesulfonyl, and the like.

Examples of the benzenesulfonyl optionally substituted with nitro represented by G³ include benzenesulfonyl that may be substituted with 1 to 3 (preferably 1) nitro groups, specifically, o-nitrobenzenesulfonyl, p-nitrobenzenesulfonyl, and the like.

G³ is preferably a chlorine atom, a fluorine atom, a bromine atom, methanesulfonyl, ethanesulfonyl, trifluoromethanesulfonyl, p-toluenesulfonyl, or p-nitrobenzenesulfonyl.

The compound represented by formula (1B) can be obtained by coupling the compound represented by formula (5) with the compound represented by formula (6). The compound represented by formula (5) and the compound represented by formula (6) are known compounds and can be produced by a known method.

This reaction can be performed, for example, in an inert solvent in the presence of a base.

Examples of inert solvents include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of bases include metal hydrides, such as sodium hydride and potassium hydride; metal hydroxides, such as potassium hydroxide and sodium hydroxide; metal carbonates, such as potassium carbonate, potassium hydrogen carbonate, sodium carbonate, sodium hydrogen carbonate, and cesium carbonate; alkyl amines, such as triethylamine and ethyldiisopropylamine; and metal alkoxides, such as sodium methoxide and potassium t-butoxide.

The amount of the compound represented by formula (6) is typically 0.5 mol or more, further 1 mol or more, preferably 0.9 to 2 mol, and more preferably 0.9 to 1.5 mol, per mol of the compound represented by formula (5).

The amount of the base for use is typically 1 mol or more, preferably 1 to 5 mol, and more preferably 1 to 2 mol, per mol of the compound represented by formula (5).

The reaction temperature is typically 30°C to a temperature higher than the boiling point of the solvent by 10°C, and preferably 80°C to a temperature higher than the boiling point of the solvent by 10°C. To facilitate the reaction, microwaves may be used. The reaction temperature in this case is, for example, 80°C to 180°C, and preferably 100°C to 180°C. The reaction time is typically 10 minutes to 48 hours, and preferably 10 minutes to 24 hours.

Additionally, the reaction of the compound represented by formula (5) with the compound represented by formula (6) can be performed by using the Buchwald reaction. For example, in the presence of a palladium catalyst, a phosphine ligand, and a base, the compound represented by formula (5) is reacted with the compound represented by formula (6) in a solvent.

Examples of palladium catalysts include divalent palladium catalysts, such as Pd(OAc)₂, PdCl₂, allyl palladium(II) chloride (dimer), bis(acetonitrile)palladium(II) dichloride, and bis(benzonitrile)palladium(II) dichloride; and zerovalent palladium catalysts, such as Pd₂(dba)₃(tris(dibenzylideneacetone) dipalladium(0)), bis(dibenzylideneacetone) palladium(0), and palladium carbon (Pd/C).

Examples of phosphine ligands include bidentate phosphine ligands, such as BINAP((2,2'-bis(diphenylphosphanyl)-1,1'-bisnaphthalene) and Xphos(2-dicyclohexylphosphino-2',4',6'-triisopropylbiphenyl).

Examples of bases include strong bases, such as t-BuONa (tert-butoxysodium).

The amount of the compound represented by formula (6) for use in this reaction is typically 0.5 mol or more, further 1 mol or more, preferably 0.9 to 2 mol, and more preferably 1 to 1.5 mol, per mol of the compound represented by formula (5).

The amount of the palladium catalyst for use is typically 0.005 to 1 mol, and preferably 0.01 to 0.2 mol, per mol of the compound represented by formula (5).

The amount of the phosphine ligand for use is typically 0.5 to 5 mol, and preferably 1 to 2 mol, per mol of the palladium catalyst.

The amount of the base for use is typically 0.5 mol or more, further 1 mol or more, and preferably 1 to 2 mol, per mol of the compound represented by formula (5).

The reaction temperature is typically 40°C to 150°C, and preferably 80°C to 110°C. The reaction time is typically 1 to 24 hours, and preferably 3 to 12 hours.

### Production Method 3

In an embodiment, the compound represented by formula (1) or (2) can be produced by the synthesis scheme illustrated in the following Reaction Formula-3. Specifically, the compound represented by formula (1C) can be produced by converting the compound represented by formula (7) into the compound represented by formula (8), reacting the compound represented by formula (8) with the compound represented by formula (4) to produce the oxime compound represented by formula (2B), and ring-closing the oxime compound represented by formula (2B). A person skilled in the art would be able to understand that the compound represented by formula (2) can be produced by using an appropriate, corresponding compound that has an optionally substituted benzene ring A, instead of the compound represented by formula (7) or (8), in the reaction illustrated in Reaction Formula-3. The compound represented by formula (7) is a known compound and can be produced by a known method. 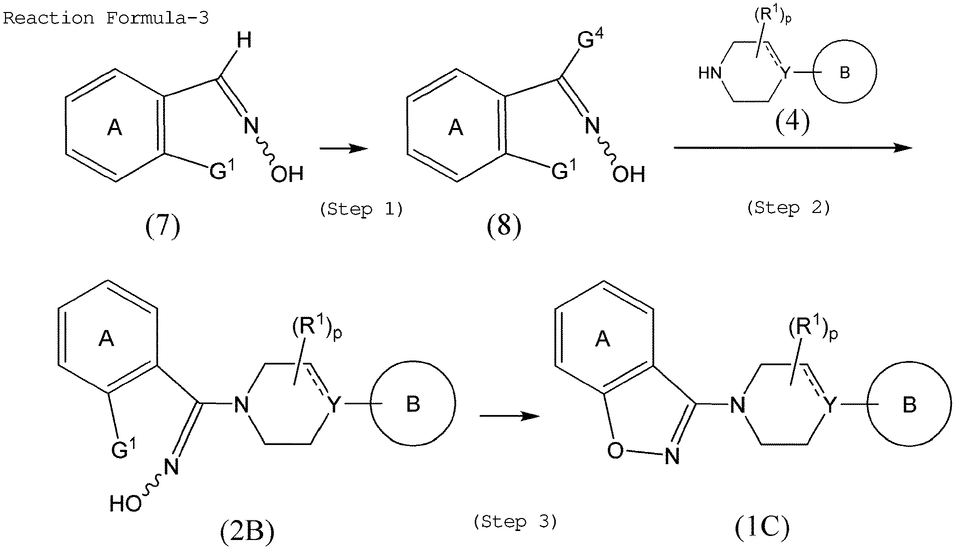 (wherein A, B, Y, R¹, p, G¹, and - - - - - - are as defined above; and G⁴ represents halogen.)

In Reaction Formula-3, A, B, Y, R¹, p, G¹, and - - - - - - are as defined above.

Examples of halogens represented by G⁴ include a chlorine atom, a fluorine atom, a bromine atom, and an iodine atom.

Step 1 (i.e., the step of converting the compound represented by formula (7) into the compound represented by formula (8)) can be performed, for example, by reacting the compound represented by formula (7) with a halogenating agent in an inert solvent.

Examples of inert solvents for use in this reaction include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of halogenating agents include typical halogenating agents, such as N-bromosuccinimide and N-chlorosuccinimide.

The amount of the halogenating agent for use is typically an equimolar amount to an excess molar amount, preferably 1- to 5-fold mol, and more preferably 1- to 2-fold mol, based on the compound represented by formula (7).

The reaction temperature is typically -30 to 150°C, preferably -10 to 100°C, and more preferably -10 to 40°C. The reaction time is typically 10 minutes to 48 hours, preferably 10 minutes to 24 hours, and more preferably 30 minutes to 18 hours.

Step 2 (i.e., the step of reacting the compound represented by formula (8) with the compound represented by formula (4) to synthesize the compound represented by formula (2B)) can be performed, for example, in an inert solvent in the presence of a base.

Examples of inert solvents for use in this reaction include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of bases include metal hydrides, such as sodium hydride and potassium hydride; metal hydroxides, such as potassium hydroxide and sodium hydroxide; metal carbonates, such as potassium carbonate, potassium hydrogen carbonate, sodium carbonate, sodium hydrogen carbonate, and cesium carbonate; alkyl amines, such as triethylamine and ethyldiisopropylamine; and metal alkoxides, such as sodium methoxide and potassium t-butoxide.

The amount of the compound represented by formula (8) for use is typically 0.5 mol or more, 0.8 mol or more, preferably 0.9 to 2 mol, and more preferably 0.9 to 1.5 mol, per mol of the compound represented by formula (4).

The amount of the base for use is typically 1 mol or more, preferably 1- to 5-fold mol, and more preferably 1- to 2-fold mol, per mol of the compound represented by formula (4).

The reaction temperature is typically -20°C to a temperature higher than the boiling point of the solvent by 10°C, and preferably 0°C to 40°C. The reaction time is typically 10 minutes to 48 hours, preferably 10 minutes to 24 hours, and more preferably 30 minutes to 18 hours.

Step 3 (i.e., the step of ring-closing the compound represented by formula (2B) to convert the compound represented by formula (2B) into the compound represented by formula (1)) can be performed, for example, in an inert solvent in the presence of a base.

The compound represented by formula (2B) exists in both forms of (E)-isomer and (Z)-isomer, which are geometric isomers. From the standpoint of less heating required in the ring-closing reaction, (E)-isomer is preferable.

Examples of inert solvents for use in this reaction include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of bases include metal hydrides, such as sodium hydride and potassium hydride; metal hydroxides, such as potassium hydroxide and sodium hydroxide; metal carbonates, such as potassium carbonate, potassium hydrogen carbonate, sodium carbonate, sodium hydrogen carbonate, and cesium carbonate; alkyl amines, such as triethylamine and ethyldiisopropylamine; and metal alkoxides, such as sodium methoxide and potassium t-butoxide.

The amount of the base for use is typically 1 mol or more, preferably 1- to 5-fold mol, and more preferably 1- to 2-fold mol, per mol of the compound represented by formula (2B).

The reaction temperature is typically 50°C to a temperature higher than the boiling point of the solvent by 10°C, and preferably 80°C to a temperature higher than the boiling point of the solvent by 10°C. To facilitate the reaction, microwaves may be used. In this case, the reaction temperature is, for example 80°C to 180°C, and preferably 100°C to 180°C. The reaction time is typically 10 minutes to 8 hours, and preferably 10 minutes to 2 hours.

The compound represented by formula (1) or (2) according to the present invention, intermediate compounds thereof, and starting material compounds thereof can be produced by the production methods described above, and can also be produced with reference to an already-known or publicly known technique at the time the present application was filed (e.g., B.R. Kiran et al., SYNTHESIS, EVALUATION OF ANALGESIC AND ANTI-INFLAMMATORY ACTIVITIES OF SUBSTITUTED 1,2-BENZOXAZOLONE AND 3-CHLORO-1,2-BENZOXAZOLE DERIVATIVES, International Journal of Pharmaceutical Sciences and Research, 2015; 6: 2918-2925) in accordance with the synthesis methods disclosed in the Examples of the present specification.

The functional groups of the starting material compounds and intermediate compounds illustrated in the reaction schemes above may optionally be protected by an appropriate protective group, using a known method, before the compounds are subjected to a reaction, and be deprotected using a known method after completion of the reaction.

The target compounds obtained in accordance with the reaction schemes above can be isolated and purified. For example, after the reaction mixture is cooled, the crude reaction product is subjected to isolation procedures, such as filtration, concentration, and extraction, in order to separate the crude reaction product; the crude reaction product is then subjected to typical purification procedures, such as column chromatography and recrystallization, thereby isolating and purifying a target compound from the reaction mixture.

The starting material compounds and the compound represented by formula (1) or (2) illustrated in the reaction schemes above include compounds in the form of solvate in which a solvent is added (e.g., a hydrate and solvate of ethanol).

When the compound represented by formula (1) or (2), the intermediate compounds obtained in the reaction schemes, or the starting material compounds have isomers, the compound represented by formula (1) or (2), the intermediate compounds obtained in the reaction schemes, or the starting material compounds include all of these isomers. The isomers include an isomer due to a double bond, a ring, or a fused ring (an E-, Z-, sis-, or trans-form); an isomer, for example, due to the presence of an asymmetric carbon (an R or S-form, α or β-form, enantiomer, or diastereomer); an optically active form that exhibits optical rotations (a D-, L-, d-, or 1-form); a polar form due to chromatographic separation (a high polar form and low polar form); an equilibrium compound; a rotamer; a mixture thereof of any proportions; and a racemic mixture (isomers such as a geometric isomer, a stereoisomer, and an optical isomer). For example, optical isomers can be separated by using various known resolution methods (e.g., optical resolution by crystallization and direct optical resolution by chromatography).

The salt of the compound represented by formula (1) or (2) includes all pharmaceutically acceptable salts. The pharmaceutically acceptable salt can be any pharmaceutically acceptable salt. Examples include alkali metal salts, such as sodium salts and potassium salts; alkaline-earth metal salts, such as calcium salts and magnesium salts; inorganic metal salts, such as zinc salts; organic base salts, such as triethylamine, triethanolamine, trihydroxymethyl amino methane, and amino acid; inorganic acid salts, such as hydrochloride, hydrobromate, sulfate, phosphate, and nitrate; and organic acid salts, such as acetate, carbonate, propionate, succinate, lactate, malate, tartrate, citrate, maleate, fumarate, methanesulfonate, p-toluenesulfonate, benzenesulfonate, and ascorbate. These salts can be produced in accordance with an ordinary method.

The variety of isomers can be isolated by a known separation method. For example, racemates can be led to sterically pure isomers by a typical optical resolution method (e.g., optical resolution by crystallization and direct optical resolution by chromatography). An optically active compound can be produced by using a suitable, optically active starting material.

The starting material compounds, intermediate compounds, and target compounds described in the reaction schemes above can be used in their suitable salt form.

In the present invention, in the compound represented by formula (1) or (2), a salt thereof, or a prodrug thereof, one or multiple atoms may be replaced by one or multiple isotopes. Examples of isotopes include deuterium (2H), tritium (3H), 13C, 14N, 180, and the like.

The pharmaceutical composition according to the present invention may be a preparation of the compound represented by formula (1) or (2), a salt thereof, or a prodrug thereof in a typical pharmaceutical composition form, and may be prepared by using the compound, a salt thereof, or a prodrug thereof and a pharmaceutically acceptable carrier. Examples of carriers include diluents and excipients, such as typically used fillers, extenders, binders, moisturizing agents, disintegrators, surfactants, and lubricants.

In the present invention, the prodrug refers to a compound that is converted into the compound represented by formula (1) or (2) through a reaction in vivo (e.g., an enzyme reaction and a reaction caused by stomach acid). For example, when the compound represented by formula (1) or (2) has carboxyl, the prodrug is a compound in which the carboxyl is converted into an ester. Examples of esters include methyl ester, ethyl ester, 1-propyl ester, 2-propyl ester, pivaloyloxy methyl ester, acetyloxymethyl ester, cyclohexyl acetyloxymethyl ester, 1-methyl cyclohexyl carbonyloxy methyl ester, ethyloxy carbonyloxy-1-ethyl ester, cyclohexyloxy carbonyloxy-1-ethyl ester, and the like.

The pharmaceutical composition according to the present invention can be selected from various dosage forms according to the purpose of treatment. Typical examples include tablets, pills, powders, liquids, suspensions, emulsions, granules, capsules, suppositories, injectable drugs (e.g., liquids and suspensions), ointments, inhalants, ear drops, and the like. Among these, preferred are preparations for oral administration, preparations for topical (preferably intra-aural) administration, and injectable drugs, and more preferred are preparations for oral administration.

The carrier for use in forming tablets can be selected from a wide range of known carriers. Examples include excipients, such as lactose, sucrose, sodium chloride, glucose, urea, starch, calcium carbonate, kaolin, and crystalline cellulose; binders, such as water, ethanol, propanol, simple syrup, a dextrose solution, a starch solution, a gelatin solution, methylcellulose, potassium phosphate, polyvinyl pyrrolidone, carboxy methylcellulose, and shellac; disintegrators, such as sodium alginate, dry starch, agar powder, laminaran powder, calcium carbonate, sodium hydrogen carbonate, a polyoxyethylene sorbitan fatty acid ester, sodium lauryl sulfate, stearic acid monoglyceride, starch, and lactose; sorbefacients, such as a quaternary ammonium base and sodium lauryl sulfate; disintegration inhibitors, such as stearin, cocoa butter, and hydrogenated oil; moisturizers, such as glycerol and starch; adsorbents, such as starch, lactose, kaolin, bentonite, and colloidal silica; lubricants, such as purified talc, stearate, boric acid powder, and polyethylene glycol; and the like.

Tablets may further optionally be formed into tablets with typical coating, such as sugarcoated tablets, gelatin-coated tablets, enteric coating tablets, film coating tablets, double-layered tablets, or multi-layered tablets.

Carriers for use in preparing pills can be selected from a wide range of known carriers. Examples include excipients, such as glucose, lactose, starch, cacao oil, hydrogenated vegetable oil, kaolin, and talc; binders, such as powdered gum arabic, powdered tragacanth, gelatin, and ethanol; and disintegrators, such as laminaran and agar.

Carriers for use in preparing suppositories can be selected from a wide range of known carriers. Examples include polyethylene glycol, cacao oil, higher alcohols, esters of higher alcohols, gelatin, and semisynthetic glycerides.

When the pharmaceutical composition according to the present invention is prepared into an injectable drug, it is preferred that the liquid agent, emulsion, or suspension be sterilized and be isotonic with blood. Diluents for use in preparing the liquid agent, emulsion, and suspension can be selected from a wide range of known diluents. Examples include water, ethanol, propylene glycol, polyoxylated isostearyl alcohols, ethoxylated isostearyl alcohols, polyoxyethylene sorbitan fatty acid esters, and the like. In the case of an injectable drug, the medical drug preparation can contain sodium chloride, glycerol, glucose, etc. in an amount sufficient to form an isotonic solution; and further can contain a typical solubilizing agent, a buffer, a soothing agent, etc., with further optionally a colorant, a preservative, an aroma component, a flavoring, a sweetener, and other medical drugs.

Ointments have forms such as a paste, cream, and gel. Examples of diluents for use in preparing an ointment of any of these forms include white petrolatum, paraffin, glycerol, cellulose derivatives, polyethylene glycol, silicone, and bentonite.

Inhalants are preparations intended to be applied to the bronchus or lungs by making a subject inhale an active ingredient in the form of aerosol. Inhalants include powdered inhalants, inhalant liquid agents, inhalant aerosol agents, and the like. Powdered inhalants refer to the preparations by which powdered solid particles in the form of aerosol are inhaled. Powdered inhalants can be typically produced by mixing an active ingredient in the form of fine particles with optional additives, such as lactose, to form a homogeneous mixture. Inhalant liquid agents refer to liquid inhalants that are applied with a device, such as a nebulizer. Inhalant liquid agents are typically produced by adding a solvent, a suitable isotonic agent, a pH adjuster, etc. to an active ingredient, and mixing them. Inhalant aerosol agents refer to metered-dose spray inhalants that spray a specific amount of an active ingredient together with a propellant that fills a container. Inhalant aerosol agents can be typically produced by adding a solvent, a suitable dispersant, stabilizer, etc. to an active ingredient to form a solution or suspension, filling a pressure-resistant container with the solution or suspension together with a liquid propellant, and attaching a metering valve to the container.

The pharmaceutical composition according to the present invention may optionally contain a colorant, a preservative, an aroma component, a flavoring, a sweetener, and other medical drugs.

The amount of the compound represented by formula (1) or (2), a salt thereof, or a prodrug thereof contained in the pharmaceutical composition according to the present invention can be any amount and can be suitably selected from a wide range of amounts. The compound according to the present invention, a salt thereof, or a prodrug thereof is present in an amount of typically 0.5 to 90 wt%, 1 to 85 wt%, and preferably 1 to 80 wt% in the pharmaceutical composition.

The method for administering the pharmaceutical composition according to the present invention is not particularly limited, and can be administered by a method according to the dosage form, patient's age, gender, disease condition, and other conditions. For example, the pharmaceutical composition according to the present invention in the form of a tablet, pill, liquid agent, suspension, emulsion, granule, or capsule can be orally administered. The pharmaceutical composition according to the present invention in the form of an injectable drug alone or in the form of an injectable drug mixed with a typical replacement fluid, such as glucose and amino acids, can be intravenously administered; or the pharmaceutical composition in the form of an injectable drug alone can be optionally administered, for example, intramuscularly, intradermally, subcutaneously, or intraperitoneally. The pharmaceutical composition according to the present invention in the form of a suppository is intrarectally administered. The pharmaceutical composition according to the present invention in the form of an inhalant is administered nasally. The pharmaceutical composition according to the present invention in the form of an ear drop is administered intra-aurally. The administration method is preferably oral administration, injection administration (including subcutaneous administration, intramuscular administration, intravenous administration, and intrathecal administration), and intra-aural administration, and more preferably oral administration.

The dose of the pharmaceutical composition according to the present invention can be determined taking into consideration the regimen, patient's age, gender, severity of disease, and other conditions. The pharmaceutical composition is administered such that the amount of the compound represented by formula (1) or (2), a salt thereof, or a prodrug thereof as an active ingredient is typically 0.01 to 100 mg, and preferably 0.1 to 50 mg, per kilogram of body weight daily in one time or in several times per day, or every two days, three days, four days, five days, six days, one week, two weeks, or four weeks. Because the dose varies depending on various conditions, a dose lower than a dose within the ranges above may be sufficient in some cases, and a dose higher than a dose within the ranges above may be necessary in other cases.

The pharmaceutical composition according to the present invention may be combined with one or more other medicinal agents to form a combination drug. Examples of other medicinal agents include medicinal agents that have an action of attenuating acute kidney injury, e.g., kidney injury caused by administration of a platinum-containing drug.

The present invention may include a method for preventing and/or treating acute kidney injury, comprising administering an effective amount of the compound represented by formula (1) or (2), a pharmaceutically acceptable salt thereof, or a prodrug thereof to a patient in need of prevention and/or treatment of acute kidney injury.

The compound represented by formula (1) or (2), a salt thereof, or a prodrug thereof has an action of preventing and/or treating acute kidney injury (in other words, an action of attenuating acute kidney injury). The compound represented by formula (1) or (2), a salt thereof, or a prodrug thereof also has an action of activating autophagy. Thus, the action of preventing and/or treating acute kidney injury may be the one achieved through the action of activating autophagy. The acute kidney injury may be prerenal, renal, or postrenal. Examples of acute kidney injury include renal ischemia-reperfusion injury associated with renal transplantation; prerenal acute kidney injury associated with decreased circulating blood volume due to massive bleeding etc.; drug-induced renal acute kidney injury caused by administration of a drug (e.g., an anticancer drug (e.g., a platinum-containing preparation) or an anti-inflammatory drug); and postrenal acute kidney injury, such as urinary tract obstruction; and the like. In particular, acute kidney injury caused by administration of a platinum-containing drug is preferable. The platinum-containing drug may be a platinum complex chemotherapeutic agent represented by cisplatin. Examples of platinum-containing drugs include cisplatin, carboplatin, nedaplatin, and oxaliplatin; preferred among these are cisplatin and nedaplatin, and particularly preferred is cisplatin.

One embodiment of the present invention includes an autophagy activator comprising the compound represented by formula (1) or (2), a salt thereof, or a prodrug thereof. The compound represented by formula (1), a salt thereof, or a prodrug thereof may have an action of activating autophagy. The compounds represented by formula (2), salts thereof, or prodrugs thereof, which are structurally similar to the compounds represented by formula (1), may have an action of activating autophagy. Thus, the compound represented by formula (1) or (2), a salt thereof, or a prodrug thereof is useful as an autophagy activator.

The compound represented by formula (1) or (2), a salt thereof, or a prodrug thereof is also useful as a pharmaceutical composition for preventing and/or treating diseases that are ameliorated by enhancing autophagy activity. Examples of the diseases that are ameliorated by enhancing autophagy activity include age-related kidney diseases (e.g., aging kidney and age-related kidney dysfunction), diabetic kidney disease, obesity-related kidney disease (e.g., obesity-related tubulopathy), Parkinson's disease, Huntington's disease, psoriasis, and the like.

The compound represented by formula (1) or (2) is not easily metabolized in the liver. Additionally, the compound is highly soluble in PBS and is thus advantageous in forming it into a preparation; additionally, due to its high membrane permeability, the compound is advantageous in bioavailability. Thus, the compound represented by formula (1) or (2) is excellent in pharmacokinetics. Thus, the compound can exhibit autophagy activity or preventive and/or therapeutic activity for kidney injury caused by administration of a platinum-containing drug for a longer period of time at smaller doses, compared with conventional autophagy activators or conventional preventive and/or therapeutic agents for kidney injury caused by administration of a platinum-containing drug.

The present invention may include a method for activating autophagy, comprising administering an effective amount of the compound represented by formula (1) or (2), a pharmaceutically acceptable salt thereof, or a prodrug thereof to a patient in need of autophagy activation treatment.

The present invention may include a method for preventing or treating a disease that is ameliorated by enhancing autophagy activity, comprising administering an effective amount of the compound represented by formula (1) or (2), a pharmaceutically acceptable salt thereof, or a prodrug thereof to a patient in need of prevention or treatment of a disease that is ameliorated by enhancing autophagy activity.

The present invention may include use of the compound represented by formula (1) or (2), a pharmaceutically acceptable salt thereof, or a prodrug thereof in the production of a preventive or therapeutic agent for a disease that is ameliorated by enhancing autophagy activity.

### Examples

The present invention is described below in more detail with reference to Reference Examples and Examples. However, the present invention is not limited to these Examples.

### Production Examples

In accordance with the methods described in Examples 1 to 43 of WO2021/079962, the following compounds were produced. Compounds represented by formula (1):
Compound 011, Compound 021, Compound 031, Compound 041, Compound 051, Compound 061, Compound 071, Compound 081, Compound 091, Compound 101, Compound 111, Compound 121, Compound 131, Compound 141, Compound 151, Compound 161, Compound 171, Compound 181, Compound 191, Compound 201, Compound 211, Compound 221, Compound 231, Compound 241, Compound 251, Compound 261, Compound 271, Compound 281, Compound 291, Compound 301, Compound 311, Compound 321, Compound 331, Compound 341, Compound 351, Compound 361, Compound 371, Compound 381, Compound 391, Compound 401, Compound 411, Compound 421, and Compound 431; and
compounds represented by formula (2):
   Compound 012, Compound 052, Compound 062, Compound 072, Compound 082, Compound 092, Compound 102, Compound 112, Compound 132, Compound 192, Compound 202, Compound 212, Compound 282, Compound 342, Compound 362, Compound 372, and Compound 412.
   5-Chloro-6-(4-(4-(trifluoromethyl)benzo[d]isoxazol-3-yl)piperazin-1-yl)nicotinic acid (Compound 011) (E)-5-Chloro-6-(4-((2-fluoro-6-(trifluoromethyl)phenyl)(hydroxyimino)methyl)piperazin-1-yl)nicotinic acid (Compound 012(E)) and its Z-isomer (Compound 012 (Z))
   5-Chloro-N-methyl-6-(4-(4-(trifluoromethyl)benzo[d]isoxazol-3-yl)piperazin-1-yl)nicotinamide (Compound 021)
   (S)-5-Chloro-6-(3-methyl-4-(4-(trifluoromethyl)benzo[d]isoxazol-3-yl)piperazin-1-yl)nicotinic acid (Compound 031)
   (R)-5-Chloro-6-(3-methyl-4-(4-(trifluoromethyl)benzo[d]isoxazol-3-yl)piperazin-1-yl)nicotinic acid (Compound 041)
   5-Chloro-6-(4-(4-(methoxy)benzo[d]isoxazol-3-yl)piperazin-1-yl)nicotinic acid (Compound 051)
   5-Chloro-6-(4-((2-fluoro-6-methoxyphenyl(hydroxyimino)methyl)piperazin-1-yl)nicotinic acid (Compound 052)
   4-Chloro-3-(4-(2-chlorophenyl)piperazin-1-yl)benzo[d]isoxazole (Compound 061)
   (E)-(4-(2-Chlorophenyl)piperazin-1-yl)(2,6-dichlorophenyl)methanone oxime (Compound 062(E)) and its Z-isomer (Compound 062(Z))
   4-Chloro-3-(4-(3-chloropyridin-2-yl)piperazin-1-yl)benzo[d]isoxazole (Compound 071)
   (E)-(4-(3-Chloropyridin-2-yl)piperazin-1-yl) (2,6-dichlorophenyl)methanone oxime (Compound 072(E)) and its Z-isomer (Compound 072(Z))
   5-Chloro-6-(4-(4-chlorobenzo[d]isoxazol-3-yl)piperazin-1-yl)nicotinic acid (Compound 081)
   5-Chloro-6-(4-((2,6-dichlorophenyl)(hydroxyimino)methyl)piperazin-1-yl)nicotinic acid (Compound 082)
   5-Chloro-6-(4-(4-chlorobenzo[d]isoxazol-3-yl)piperazin-1-yl)N,N-dimethylnicotinamide (Compound 091)
   (E)-5-Chloro-6-(4-((2,6-dichlorophenyl)(hydroxyimino)methyl)piperazin-1-yl)-N,N-dimethylnicotinamide (Compound 092(E)) and its Z-isomer (Compound 092 (Z))
   3-Chloro-4-(4-(4-chlorobenzo[d]isoxazol-3-yl)piperazin-1-yl)benzoic acid (Compound 101)
   3-Chloro-4-(4-((2,6-dichlorophenyl)(hydroxyimino)methyl)piperazin-1-yl)benzoic acid methyl ester (Compound 102)
   5-Chloro-3-(4-(3-chloropyridin-2-yl)piperazin-1-yl)benzo[d]isoxazole (Compound 111)
   (E)-(4-(3-Chloropyridin-2-yl)piperazin-1-yl) (2,5-dichlorophenyl)methanone oxime (Compound 112(E)) and its Z-isomer (Compound 112(Z))
   5-Chloro-6-(4-(5-chlorobenzo[d]isoxazol-3-yl)piperazin-1-yl)nicotinic acid (Compound 121)
   5-Chloro-6-(4-(5-(trifluoromethyl)benzo[d]isoxazol-3-yl)piperazin-1-yl)nicotinic acid (Compound 131)
   5-Chloro-6-(4-((2-chloro-5-(trifluoromethyl)phenyl)(hydroxyimino)methyl)piperazin-1-yl)nicotinic acid (Compound 132)
   3-(4-(Pyridin-2-yl)piperazin-1-yl)benzo[d]isoxazole (Compound 141)
   3-(4-(2-Chlorophenyl)piperazin-1-yl)benzo[d]isoxazole (Compound 151)
   3-(4-(2,3-Dimethylphenyl)piperazin-1-yl)benzo[d]isoxazole (Compound 161)
   3-(4-(2,4-Dichlorophenyl)piperazin-1-yl)benzo[d]isoxazole (Compound 171)
   3-(4-(3-Chloropyridin-2-yl)piperazin-1-yl)benzo[d]isothiazole (Compound 181)
   5-Chloro-6-(8-(4-(trifluoromethyl)benzo[d]isoxazol-3-yl)-3,8-diazabicyclo[3,2,1]octan-3-yl)nicotinic acid (Compound 191)
   5-Chloro-6-(8-((2-fluoro-6-(trifluoromethyl)phenyl(hydroxyimino)methyl)-3,8-diazabicyclo[3,2,1]octan-3-yl)nicotinic acid (Compound 192)
   7-Chloro-3-(4-(2-chlorophenyl)piperazin-1-yl)benzo[d]isoxazole (Compound 201)
   (E)-(4-(2-Chlorophenyl)piperazin-1-yl)(2,3-dichlorophenyl)methanone oxime (Compound 202(E)) and its Z-isomer (Compound 202(Z))
   6-Chloro-3-(4-(2-chlorophenyl)piperazin-1-yl)benzo[d]isoxazole (Compound 211)
   (E)-(4-(2-Chlorophenyl)piperazin-1-yl) (2,4-dichlorophenyl)methanone oxime (Compound 212(E)) and its Z-isomer (Compound 212(Z))
   5-Chloro-6-(4-(4-chlorobenzo[d]isoxazol-3-yl)piperazin-1-yl)pyridin-3-yl)(morpholino)methanone (Compound 221)
   5-Chloro-6-(4-(4-chlorobenzo[d]isoxazol-3-yl)piperazin-1-yl)-N-(methylsulfonyl)nicotinamide (Compound 231)
   Methyl 5-chloro-6-(4-(4-(trifluoromethyl)benzo[d]isoxazol-3-yl)piperazin-1-yl)nicotinate (Compound 241)
   (5-Chloro-6-(4-(4-(trifluoromethyl)benzo[d]isoxazol-3-yl)piperazin-1-yl)pyridin-3-yl)methanol (Compound 251)
   3-(4-(3-Methylpyridin-2-yl)piperazin-1-yl)-4-(trifluoromethyl)benzo[d]isoxazole (Compound 261)
   4-Methoxy-3-(4-(2-chlorophenyl)piperazin-1-yl)benzo[d]isoxazole (Compound 271)
   1-(1-(4-Trifluoromethyl)benzo[d]isoxazol-3-yl)piperidin-4-yl)-1,3-dihydro-2H-benzo[d]imidazol-2-one (Compound 281)
   1-(1-((2-Fluoro-6-(trifluoromethyl)phenyl(hydroxyimino)methyl)piperidin-4-yl)-1,3-dihydro-2H-benzo[d]imidazol-2-one (Compound 282
   2-(2-Oxo-3-(1-(4-(trifluoromethyl)benzo[d]isoxazol-3-yl)piperidin-4-yl)-2,3-dihydro-1H-benzo[d]imidazol-1-yl) acetic acid tert-butyl ester (Compound 291)
   2-(2-Oxo-3-(1-(4-(trifluoromethyl)benzo[d]isoxazol-3-yl)piperidin-4-yl)-2,3-dihydro-1H-benzo[d]imidazol-1-yl) acetic acid (Compound 301)
   3-(4-(2-Chloropyridin-3-yl)piperazin-1-yl)benzo[d]isoxazole (Compound 311)
   3-(4-(3-Chloropyridin-2-yl)piperazin-1-yl)benzo[d]isoxazole (Compound 321)
   3-(4-(3,5-Dichlorophenyl)piperazin-1-yl)benzo[d]isoxazole (Compound 331)
   Methyl 5-chloro-6-(1-(4-(trifluoromethyl)benzo[d]isoxazol-3-yl)piperidin-4-yl)nicotinate (Compound 341)
   Methyl 5-chloro-6-(1-((2-fluoro-6-(trifluoromethyl)phenyl)(hydroxyimino)methyl)piperidin-4-yl)nicotinate (Compound 342)
   5-Chloro-6-(1-(4-(trifluoromethyl)benzo[d]isoxazol-3-yl)piperidin-4-yl)nicotinic acid (Compound 351)
   3-(4-(2-Chlorophenyl)-3,6-dihydropyridin-1(2H)-yl)-4-(trifluoromethyl)benzo[d]isoxazole (Compound 361)
   E-(4-(2-Chlorophenyl)-3,6-dihydropyridin-1(2H)-yl) (2-fluoro-6-(trifluoromethyl)phenyl)methanone oxime (Compound 362(E)) and its Z-isomer (Compound 362(Z))
   Ethyl 5-Chloro-6-(2-oxo-4-(4-(trifluoromethyl)benzo[d]isoxazol-3-yl)piperazin-1-yl)nicotinate (Compound 371)
   Ethyl (Z)-5-chloro-6-(4-((2-fluoro-6-(trifluoromethyl)phenyl)(hydroxyimino)methyl)-2-oxopiperazin-1-yl)nicotinate (Compound 372(Z))
   5-Chloro-6-(2-oxo-4-(4-(trifluoromethyl)benzo[d]isoxazol-3-yl)piperazin-1-yl)nicotinic acid (Compound 381)
   Methyl 3-chloro-4-(1-(4-(trifluoromethyl)benzo[d]isoxazol-3-yl)-1,2,3,6-tetrahydropyridin-4-yl)benzoate (Compound 391)
   Methyl 3-chloro-4-(1-(4-(trifluoromethyl)benzo[d]isoxazol-3-yl)-1,2,3,6-tetrahydropyridin-4-yl)benzoic acid (Compound 401)
   3-(4-(2-Chlorophenyl)piperazin-1-yl)-4-iodobenzo[d]isoxazole (Compound 411)
   (E)-(4-(2-Chlorophenyl)piperazin-1-yl) (2-fluoro-6-iodophenyl)methanone oxime (Compound 412(E)) and its Z isomer (Compound 412(Z))
   3-(4-(2-Chlorophenyl)piperazin-1-yl)-4-cyanobenzo[d]isoxazole (Compound 421)
   3-(4-(2-Chlorophenyl)piperazin-1-yl)benzo[d]isoxazole-4-carboxyamide (Compound 431)

In the following Test Examples, quantitative analysis of histological staining was all performed by two nephrology specialists by blindly evaluating at least 10 high-magnification fields of each tissue.

### Test Example 1: Action of Compound 011 on Kidney Injury Induced by Cisplatin Administration

### Test Methods

Wild-type male mice were used as test animals. The mice had free access to water and standard mouse chow.

Cisplatin (Sigma-Aldrich) was dissolved in physiological saline at a concentration of 1 mg/mL to prepare a cisplatin solution.

The cisplatin solution was intraperitoneally administered to 8-week-old mice in such an amount that the cisplatin dose was 15 mg/kg body weight. The same amount of physiological saline was intraperitoneally administered to the control group.

A sample liquid was prepared by suspending Compound 011 (test compound) in PBS.

The sample liquid was orally administered in such an amount that the dose of the test compound was 10 mg/kg body weight for 3 consecutive days from the previous day of the day of the cisplatin administration. The same amount of PBS was orally administered to the control group.

Three days after the cisplatin administration, the mice were dissected under anesthesia, and the blood and kidneys were collected.

Additionally, the mice that were not administered with cisplatin were treated in a similar manner, and the blood and kidneys were collected. The mice were thus divided into four groups.

### Histological Analysis

The mice were perfused transcardially with PBS (pH of 7.4). The collected kidneys were sliced, post-fixed in 4% PFA (paraformaldehyde), and embedded in paraffin or frozen in OCT compound to obtain section samples of renal cortex.

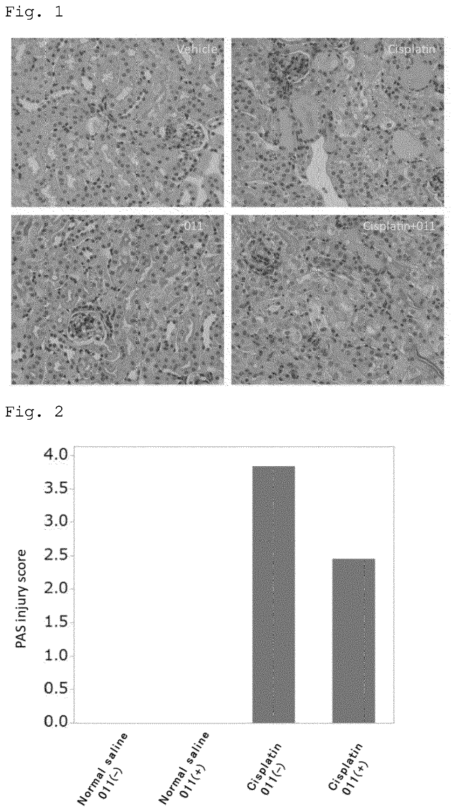

The section samples of the four groups were stained with PAS (Periodic acid-Schiff) and observed under an optical microscope (400x) (Fig. 1).

Necrosis of renal tubular cells, flattening and dilatation of the renal tubular epithelium, and cast formation were observed as a result of cisplatin administration, indicating that the renal tubules were significantly damaged (the upper-right image in Fig. 1 (the cisplatin administration group)). The administration of the test compound clearly attenuated renal tubular injury caused by cisplatin (the lower-right image in Fig. 1 (the cisplatin and test compound administration group)). The test compound administration group (the lower-left image in Fig. 1 (the test compound administration group)) showed no apparent change compared with the vehicle administration group (the upper-left image in Fig. 1 (the vehicle administration group)).

The renal tubular injury was quantitatively evaluated using PAS-stained section samples. More specifically, the section samples of the four groups were observed with at least 10 high-magnification fields (200x), and the scores of renal tubular injury (PAS injury scores, average values) were evaluated on a 10-scale grade based on the percentage of renal tubular injury. The renal tubular injury was defined to be cellular necrosis, tubular dilatation, and cast formation.

The results are shown in Fig. 2. In the bar graph of Fig. 2, "Normal saline" represents a group administered with physiological saline without cisplatin (vehicle), and "Cisplatin" represents a group administered with physiological saline in which cisplatin was dissolved. This also applies to the bar graphs shown in other figures.

Although renal tubular injury significantly increased with the cisplatin administration, renal tubular injury was clearly attenuated with the test compound administration.

### Biochemical Analysis

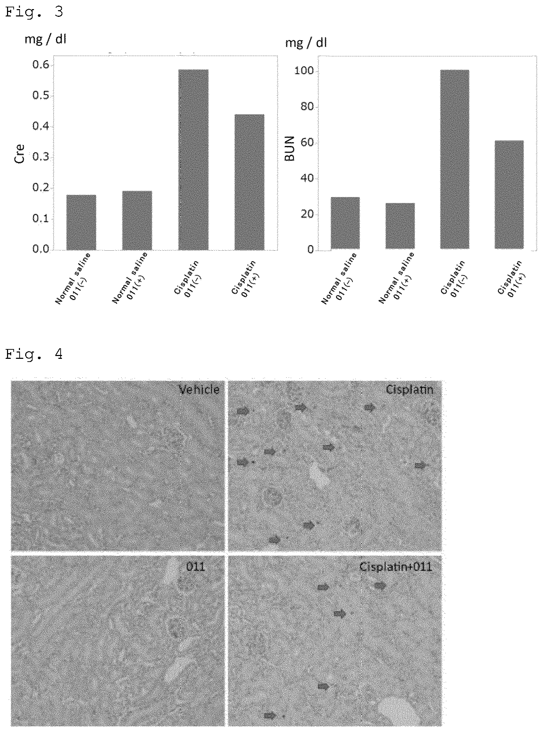

Plasma was obtained from the blood collected from the mice to measure the creatinine concentration and the urea nitrogen concentration (n = 2 to 4 for each group). The creatinine concentration was measured by using a BioAssay Systems' Creatinine Assay Kit (DICT-500), and the urea nitrogen concentration was measured by using a Wako BUN-test. Fig. 3 shows the results. In Fig. 3, "Normal saline" represents a group administered with physiological saline without cisplatin, and "Cisplatin" represents a group administered with physiological saline in which cisplatin was dissolved. Additionally, in Fig. 3, "Cre" represents the creatinine concentration and "BUN" represents the serum urea nitrogen concentration.

The administration of the test compound normally causes little or no change in the creatinine concentration and the serum urea nitrogen concentration. On the other hand, the administration of cisplatin markedly increases the creatinine concentration and the serum urea nitrogen concentration. However, such increases were clearly suppressed by the administration of the test compound. This confirmed that the test compound can attenuate the deterioration of renal function caused by cisplatin.

### Test Example 2: Action of Compound 011 on (1) DNA Damage Induced by Cisplatin Administration and (2) the Amount of Organelles Degraded by Autophagy after Cisplatin Administration

### Histological Analysis

The kidneys of mice were collected as in Test Example 1. The kidneys were subjected to autoclaving at 120°C for 10 minutes in a 0.01 mmol/L citrate buffer (pH of 6.0) for antigen inactivation and to blocking with 1.5% bovine serum albumin in PBS for 60 minutes. Subsequently, paraffin embedding was performed to prepare section samples of renal cortex of the four groups.

The sections were incubated overnight at 4°C with p53 or p62 primary antibodies.

For each group (n = 2 to 4), the number of p53-positive cells or p62-positive dots were counted in fields at a magnification of 200x (p53) or 400x (p62) to calculate the average values.

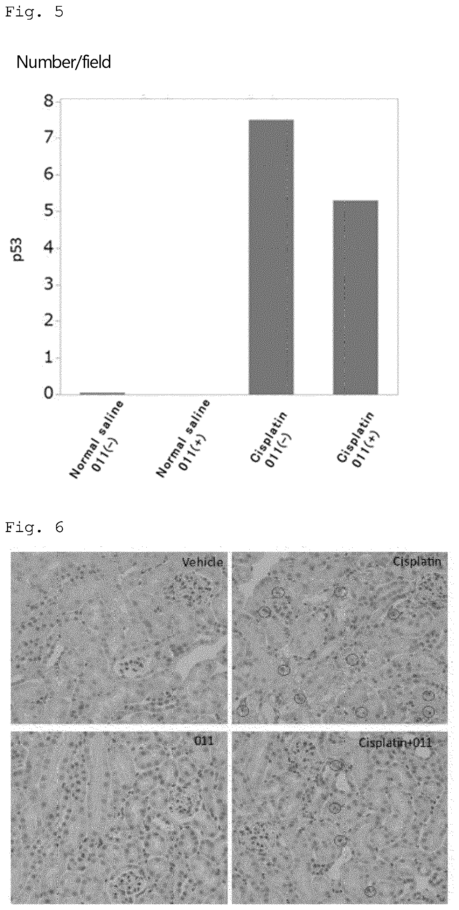

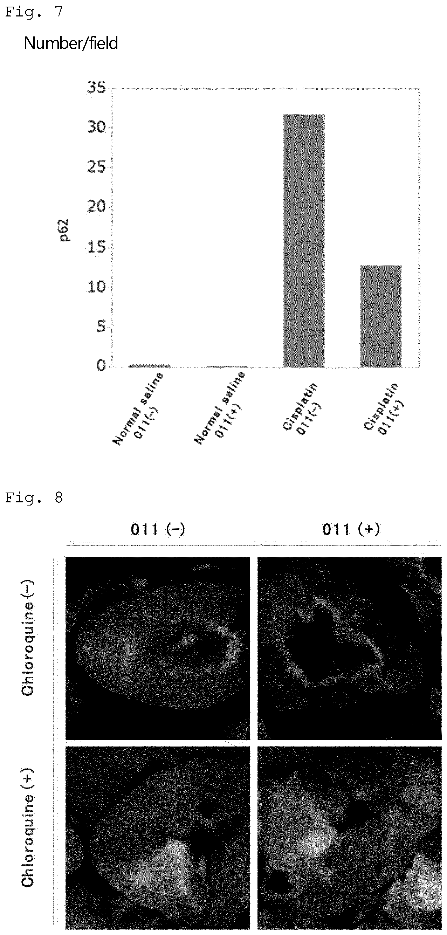

Fig. 4 shows microscopic images (200x) obtained using p53. Fig. 5 shows the number of p53-positive cells (average values). Fig. 6 shows microscopic images (400x) obtained using p62. Fig. 7 shows the number of p62-positive dots (average values). The arrows in Fig. 4 indicate p53-positive cells. In Fig. 6, p62 positive dots are enclosed in circles.

P53 is known as a cancer suppressor gene and functions in cell cycle arrest, apoptosis, DNA repair, etc., depending on the degree of cellular stress. The appearance of p53 is assessed as severe damage to nuclear DNA. p62 is a substrate that is selectively degraded by autophagy. The appearance of p62 is assessed as a state in which autophagy is unable to sufficiently degrade the substrate, i.e., an autophagy-deficient state. The administration of the test compound clearly reduced p53 and p62, which appear when cisplatin is administered. These results confirmed that the administration of the test compound suppresses DNA damage and suppresses autophagy impairment.

### Test Example 3: Action of Compound 011 on Autophagy Flux

The test animals were 8-week-old male autophagy-monitoring mice (GFP-LC3 transgenic mice). The mice had free access to water and standard mouse chow.

The cisplatin solution was intraperitoneally administered to the mice in such an amount that the cisplatin dose was 15 mg/kg body weight.

The test compound (compound 011) was suspended in PBS to prepare a sample liquid.

The sample liquid was orally administered in such an amount that the dose of the test compound was 10 mg/kg body weight for 3 consecutive days from the previous day of the day of the cisplatin administration.

Two days after the cisplatin administration, the mice were dissected under anesthesia and the kidneys were collected.

Six hours before anesthetization of the mice, a chloroquine solution was administered intraperitoneally as a lysosomal inhibitor at 50 µg/g body weight. Separately, a group in which the chloroquine solution was not administered was also prepared. The chloroquine solution was prepared by dissolving chloroquine (Sigma-Aldrich) in distilled water at a concentration of 5 mg/mL.

### Histological Analysis

The autophagy flux was evaluated by quantifying GFP-LC3 dots (GFP-positive dots).

The kidney sections were immunostained with LRP2/Megalin (red), which is a marker for proximal tubular cells, and contrast-stained with DAPI (blue).

The number of GFP-LC3 dots (green) per proximal tubule in each group (n = 5) was quantified in 10 high-magnification fields (600x), and the average values were used for evaluation.

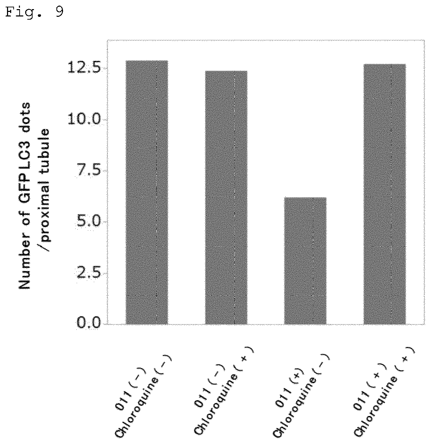

Fig. 8 shows microscopic images (×2400) of the sections, and Fig. 9 shows the number of GFP-LC3 dots. The green color in the microscopic images reflects autophagosomes that have not been degraded intracellularly.

LC3 is a protein that binds to autophagosome membranes. A small number of LC3 dots, i.e., autophagosomes (LC3-bound autophagosome membranes), appear in renal tubules in a steady state. LC3 dots increase when autophagy is induced by various stresses. In GFP-LC3 transgenic mice, an increase in GFP-LC3 dots has typically been considered to mean an increase in autophagosomes and indicate autophagy activation. However, methods for accurately evaluating autophagy activity in disease pathologies are still complicated and require caution in interpretation. For example, an increase in the number of autophagosomes or LC3 does not unconditionally indicate autophagy activation, but may possibly indicate a block (bottleneck) in a later step of autophagy, such as dysfunction of lysosomes, where substrate degradation takes place.

To address the above issue, the present inventors previously established a method for evaluating autophagy activity by performing administration of chloroquine, which is a lysosomal inhibitor, and comparing the number of GFP-LC3 dots between with and without the administration (NPL 5-7). Specifically, GFP-LC3 dots significantly increase with chloroquine administration when autophagy activity is high. In contrast, when autophagy activity is low or stagnant, GFP-LC3 dots do not increase even with chloroquine administration, compared with before the administration.

When cisplatin was administered to GFP-LC3 transgenic mice, increased GFP-LC3 dots (increased autophagosomes) were observed (the upper-left image in Fig. 8,); however, no increase in GFP-LC3 dots was observed even when chloroquine was administered on the second day of cisplatin administration (the lower-left image in Fig. 8). This indicates that although autophagy was induced by the cisplatin administration, autophagy flux had already stagnated. On the other hand, the test compound administration group (the upper-right image in Fig. 8) showed a decrease in GFP-LC3 dots 2 days after the cisplatin administration. This suggests that the autophagy flux stagnation had been released. In fact, GFP-LC3 dots were significantly increased in the test compound and chloroquine administration group (the lower-right image in Fig. 8), indicating that autophagy flux stagnation was released and autophagy was enhanced.

The above results confirmed that the administration of the test compound releases the stagnation of autophagy flux caused by cisplatin and enhances autophagy.

### Test Example 4: Action of Compound 011 on Autophagy Activity in Lung Tumor Cells

### Western Blotting

A549 cells (1.0 × 10⁵ cells/well), i.e., human lung tumor cells, were seeded in a 6-well plate containing DMEM medium and cultured for 1 day, and the cells were washed with PBS.

A DMEM medium containing Bafilomycin A1 (final concentration: 250 nM; also referred to as "Baf") (Baf(+)) and a DMEM medium not containing Baf (Baf(-)) were prepared. Additionally, a Baf-containing DMEM medium with DMSO or Compound 011(the test compound) and a Baf-free DMEM medium with DMSO or Compound 011(the test compound) were also prepared, thus preparing a total of six different media. The final concentration of DMSO was 0.1% and the final concentration of the test compound was 10 pM. Separately, an Earle's balanced salt solution (EBSS; starvation) containing Baf and an EBSS not containing Baf were also prepared. 3 mL of one of the six types of media and two types of EBSSs was added to each well containing washed cells, followed by culturing for 6 hours. After washing the cells three times with PBS, RIPA buffer was added at 4°C, and the cells were allowed to stand on ice for 30 minute. The cells were then quickly and carefully collected from the wells. After the cells were collected, centrifugation (15000 rpm, 4°C, 30 min) was performed, and the supernatant was collected to obtain a protein solution.

The protein solution was denatured by adding a Sample buffer, electrophoresed on a 15% polyacrylamide gel, and then transferred to a nitrocellulose membrane. Blocking was performed in a blocking buffer. Anti-LC3 (diluted 1000-fold) and anti-β-actin (diluted 10000-fold) were used as primary antibodies, and anti-rabbit (diluted 2000-fold) and anti-mouse (diluted 2000-fold) were used as secondary antibodies, and detection was performed with luminescent reagents (ELC Western Blotting Detection Reagents (Amersham Bioscience)). Image J was used for the analysis.

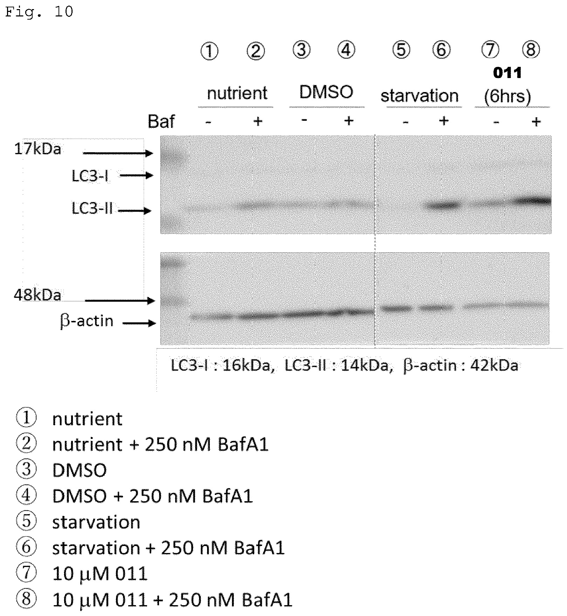

Fig. 10 shows the results of electrophoresis. In Fig. 10, "nutrient" represents a group of DMEM medium not containing DMSO and the test compound, "DMSO" represents a group of DMEM medium containing DMSO, "starvation" represents an EBSS group, and "011" represents a test compound group.

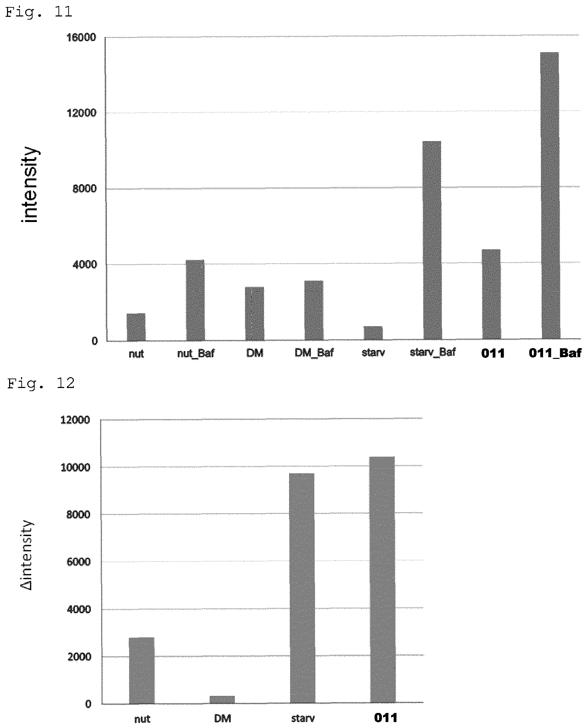

Fig. 11 shows the LC3-II protein level (luminescence intensity) of each group. Fig. 12 shows the difference (Δ) obtained by subtracting the protein level without Baf treatment from the protein level with Baf treatment for each group.

LC3-II protein is a marker for autophagy and increases when autophagy is induced. The increased LC3-II is degraded partly in the process of autophagy. Baf inhibits such degradation. Thus, the autophagy hyperactivity can be evaluated based on the percentage of increased LC3-II level with the Baf treatment, relative to the LC3-II level without the Baf treatment. As shown in Fig. 12, the test compound showed a significant increase in LC3-II with the Baf treatment, and the degree of the increase is comparable to that of starvation, confirming that the test compound has an autophagy-inducing effect.

It has been observed or reported that stagnation of autophagy flux also occurs in acute kidney injury due to renal ischemia-reperfusion injury, unilateral ureteral obstruction (urinary tract obstruction), etc., despite the protective nature of autophagy. In the Test Examples in the present application, the test compound has been confirmed to have an autophagy-enhancing effect. Based on these facts, the test compound is expected to be effective against acute kidney injury.

## Claims

1. A pharmaceutical composition for preventing and/or treating acute kidney injury, comprising a compound represented by formula (1), a salt thereof, or a prodrug thereof: 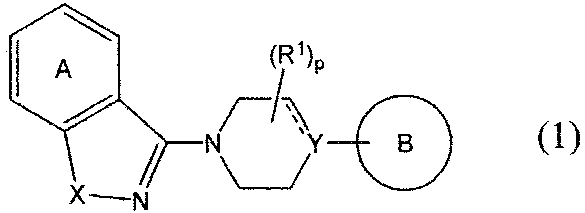 wherein
A is an optionally substituted benzene ring;
B is an optionally substituted aryl or an optionally substituted heteroaryl;
X is an oxygen atom or a sulfur atom;
Y is a nitrogen atom or a carbon atom;
- - - - - - of - - - - -Y is a single or double bond when Y is a carbon atom, or
- - - - - - of - - - - -Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

2. The pharmaceutical composition according to claim 1, wherein in formula (1),
B is an optionally substituted monocyclic aryl or an optionally substituted monocyclic or bicyclic nitrogen-containing heteroaryl.

3. The pharmaceutical composition according to claim 1 or 2, wherein in formula (1),
A is a benzene ring optionally substituted with at least one substituent selected from the group consisting of the following A-1 to A-16:
A-1) halogen,
A-2) hydroxyl,
A-3) nitro,
A-4) cyano,
A-5) carboxyl,
A-6) optionally substituted amino,
A-7) optionally substituted cyclic amino,
A-8) optionally substituted lower alkyl,
A-9) optionally substituted lower alkoxy,
A-10) lower alkoxycarbonyl,
A-11) lower alkylsulfonyl,
A-12) carbamoyl optionally substituted with lower alkyl or lower alkylsulfonyl,
A-13) optionally substituted cyclic aminocarbonyl,
A-14) sulfamoyl optionally substituted with lower alkyl,
A-15) optionally substituted cyclic aminosulfonyl, and
A-16) tetrazolyl.

4. The pharmaceutical composition according to any one of claims 1 to 3, wherein in formula (1),
B is a monocyclic aryl or a monocyclic or bicyclic heteroaryl, the monocyclic aryl is optionally substituted with at least one substituent selected from the group consisting of the following B-1 to B-16, and
the monocyclic or bicyclic heteroaryl is optionally substituted with at least one substituent selected from the group consisting of the following B-1 to B-17:
B-1) halogen,
B-2) hydroxyl,
B-3) nitro,
B-4) cyano,
B-5) carboxyl
B-6) optionally substituted amino,
B-7) optionally substituted cyclic amino,
B-8) optionally substituted lower alkyl,
B-9) optionally substituted lower alkoxy,
B-10) lower alkoxycarbonyl,
B-11) lower alkylsulfonyl,
B-12) carbamoyl optionally substituted with lower alkyl or lower alkylsulfonyl,
B-13) optionally substituted cyclic aminocarbonyl,
B-14) sulfamoyl optionally substituted with lower alkyl,
B-15) optionally substituted cyclic aminosulfonyl,
B-16) tetrazolyl, and
B-17) oxo.

5. The pharmaceutical composition according to any one of claims 1 to 4, wherein in formula (1), the benzisoxazole or benzisothiazole skeleton is substituted at the 4-position.

6. The pharmaceutical composition according to any one of claims 1 to 3, wherein in formula (1),
B is substituted pyridyl or substituted phenyl, and at least one of the carbon atoms in ortho-positions with respect to the Y-bound carbon atom on the pyridine or benzene ring is substituted.

7. The pharmaceutical composition according to any one of claims 1 to 4, wherein in formula (1),
A is a benzene ring optionally substituted with at least one substituent selected from the group consisting of halogen, lower alkoxy, and optionally halogen-substituted lower alkyl;
B is pyridyl or phenyl each optionally substituted with at least one substituent selected from the group consisting of the following B-1, B-5, B-8, B-10, B-12, and B-13:
B-1) halogen,
B-5) carboxyl,
B-8) optionally substituted lower alkyl,
B-10) lower alkoxycarbonyl,
B-12) carbamoyl optionally substituted with lower alkyl or lower alkylsulfonyl, and
B-13) optionally substituted cyclic aminocarbonyl; and
each R¹ independently represents C₁-C₃ alkyl, or
two R¹s are bound to each other to form a methylene group, a dimethylene group, or a trimethylene group, or
(R¹)ₚ is oxo.

8. The pharmaceutical composition according to any one of claims 1 to 4 and 7, wherein the compound represented by formula (1) is a compound represented by formula (1A): 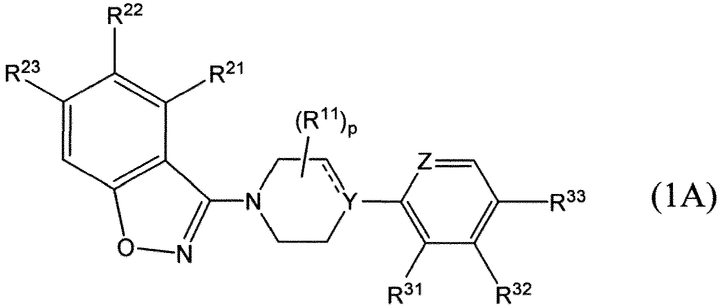 wherein
Z is a nitrogen atom or CH;
Y is a nitrogen atom or a carbon atom;
- - - - - - of - - - - -Y is a single or double bond when Y is a carbon atom, or - - - - - - of - - - - -Y is a single bond when Y is a nitrogen atom;
each R¹¹ independently represents methyl or ethyl, or
two R¹¹s may be bound to each other to form a crosslinked structure by methylene, dimethylene, or trimethylene;
p is 0, 1, or 2; or
(R¹¹)ₚ is oxo;
R²¹, R²², and R²³ independently represent a hydrogen atom, halogen, carbamoyl, or trifluoromethyl; and
R³¹, R³², and R³³ independently represent a hydrogen atom, halogen, halogen-substituted lower alkyl, methyl, carboxyl, lower alkoxycarbonyl, monomethylaminocarbonyl, or dimethylaminocarbonyl.

9. The pharmaceutical composition according to claim 8, wherein in formula (1A),
R²¹ is a chlorine atom or trifluoromethyl,
R²² and R²³ are each a hydrogen atom,
R³¹ is a chlorine atom,
R³² is a hydrogen atom, and
R³³ is a hydrogen atom, carboxyl, or lower alkoxycarbonyl.

10. The pharmaceutical composition according to any one of claims 1 to 4, wherein the compound represented by formula (1) is Compound 011, Compound 021, Compound 031, Compound 041, Compound 061, Compound 071, Compound 081, Compound 091, Compound 101, Compound 111, Compound 121, Compound 131, Compound 141, Compound 151, Compound 161, Compound 171, Compound 191, Compound 221, Compound 281, Compound 311, Compound 321, Compound 331, Compound 341, Compound 351, Compound 361, Compound 371, Compound 381, Compound 391, Compound 401, or Compound 431, each represented by any of the following structures:

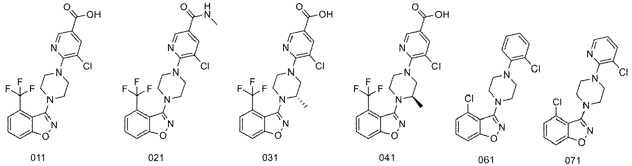

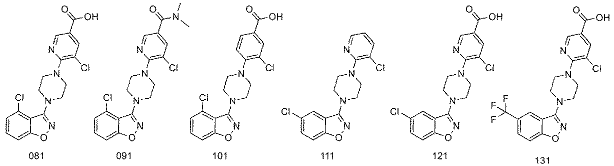

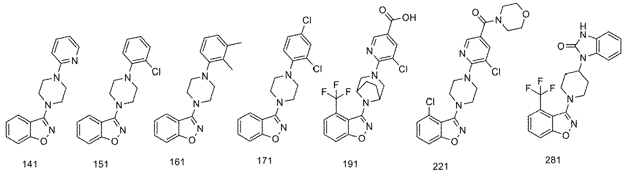

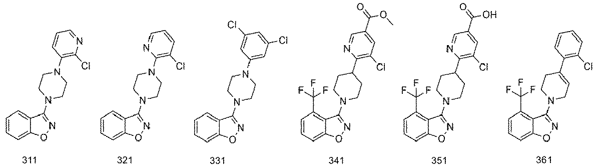

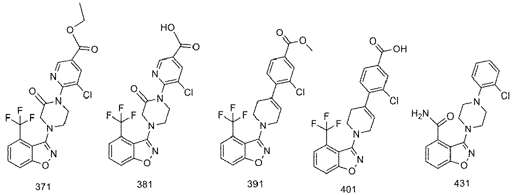

11. A pharmaceutical composition for preventing and/or treating acute kidney injury, comprising a compound represented by formula (2), a salt thereof, or a prodrug thereof: 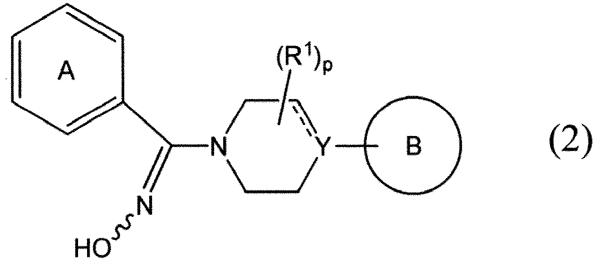 wherein
A is an optionally substituted benzene ring;
B is an optionally substituted aryl or an optionally substituted heteroaryl;
Y is a nitrogen atom or a carbon atom;
- - - - - - of - - - - -Y is a single or double bond when Y is a carbon atom, or
- - - - - - of - - - - -Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

12. The pharmaceutical composition according to any one of claims 1 to 11, which is for oral administration.

13. The pharmaceutical composition according to any one of claims 1 to 12, wherein the acute kidney injury is kidney injury caused by administration of a platinum-containing drug.

14. The pharmaceutical composition according to claim 13, wherein the platinum-containing drug is cisplatin.

15. An autophagy activator comprising a compound represented by formula (1), a salt thereof, or a prodrug thereof: 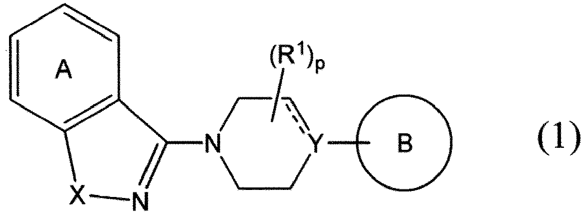 wherein
A is an optionally substituted benzene ring;
B is an optionally substituted aryl or an optionally substituted heteroaryl;
X is an oxygen atom or a sulfur atom;
Y is a nitrogen atom or a carbon atom;
- - - - - - of - - - - -Y is a single or double bond when Y is a carbon atom, or
- - - - - - of - - - - -Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

16. An autophagy activator comprising a compound represented by formula (2), a salt thereof, or a prodrug thereof: 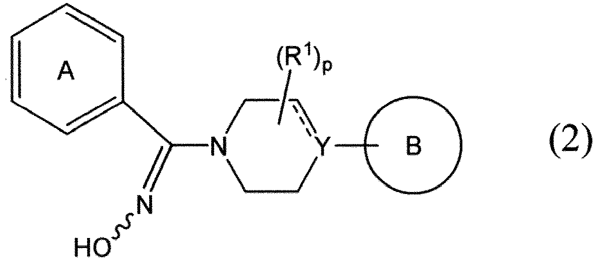 wherein
A is an optionally substituted benzene ring;
B is an optionally substituted aryl or an optionally substituted heteroaryl;
Y is a nitrogen atom or a carbon atom;
- - - - - - of - - - - -Y is a single or double bond when Y is a carbon atom, or
- - - - - - of - - - - -Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.
